# FASCICULE DE BREVET EUROPEEN

(11) **EP 1 147 187 B1**
(45) Date de publication et mention de la délivrance du brevet: **27.06.2012**
(21) Numéro de dépôt: 00900645.3
(22) Date de dépôt: 21.01.2000
(51) Int. Cl.: C12N 15/11, C07K 14/15, C12Q 1/68

(54) **PROCÉDÉ DE DÉTECTION D'UN FRAGMENT NUCLÉIQUE ENDOGÈNE ASSOCIÉ À UNE MALADIE AUTO-IMMUNE**
VERFAHREN ZUR ERKENNUNG EINES NUKLEINSÄUREFRAGMENTS VERBUNDEN MIT EINER AUTOIMMUNERKRANKUNG.
PROCESS FOR THE DETECTION OF AN ENDOGENOUS NUCLEIC ACID FRAGMENT ASSOCIATED WITH AN AUTOIMMUNE DISEASE

(30) Priorité: 21.01.1999 FR 9900888
(43) Date de publication de la demande: 24.10.2001
(73) Titulaire: BIO MERIEUX, 69280 Marcy l'Etoile (FR)
(72) Inventeur: PARANHOS-BACCALA, Glaucia, 69003 Lyon (FR); MALLET, François, 69100 Villeurbanne (FR); VOISSET, Cécile, London WC1H 9QW (GB)
(74) Mandataire: Guerre, Dominique
(86) Numéro de dépôt international: PCT/FR2000/000144
(87) Numéro de publication internationale: WO 2000/043521

(56) Documents cités:
- EP-A- 0 731 168
- WO-A-93/07259
- WO-A-93/20188
- WO-A-94/28138
- WO-A-95/21256
- WO-A-98/23755
- WO-A-99/02696
- FR-A- 2 737 500
- FR-A- 2 765 588
- PERRON H ET AL: "MOLECULAR IDENTIFICATION OF A NOVEL RETROVIRUS REPEATEDLY ISOLATED FROM PATIENTS WITH MULTIPLE SCLEROSIS" PROCEEDINGS OF THE NATIONAL ACADEMY OF SCIENCES OF USA,US,NATIONAL ACADEMY OF SCIENCE. WASHINGTON, vol. 94, 1 juillet 1997 (1997-07-01), pages 7583-7588, XP002062853 ISSN: 0027-8424
- PERRON H ET AL: "IN VITRO TRANSMISSION AND ANTIGENICITY OF A RETROVIRUS ISOLATED FROM A MULTIPLE SCLEROSIS PATIENT" RESEARCH IN VIROLOGY,FR,ELSEVIER, PARIS, vol. 143, no. 5, 1 janvier 1992 (1992-01-01), pages 337-350, XP000569296 ISSN: 0923-2516
- KOMURIAN-PRADEL F. ET A.: "Molecular identification and characterization of a novel retrovirus (MSRV) isolated from patients with multiple sclerosis (MS)" JOURNAL OF NEUROIMMUNOLOGY, vol. 90, no. 1, septembre 1998 (1998-09), page 75 XP000892882
- KOMURIAN-PRADEL F. ET AL.: "Molecular cloning and characterization of MSRV-related sequences associated with retrovirus-like particles" VIROLOGY, vol. 260, no. 1, juillet 1999 (1999-07), page 1-9 XP000891352 ORLANDO US

## Description

La présente invention se rapporte à un fragment nucléique endogène de type rétroviral intégré à l'ADN du génome humain.

Les rétrovirus sont des virus à ARN qui se répliquent par un processus dit de transcription inverse, médié par une ADN polymérase ARN dépendante dénommée reverse transcriptase (RT), codée par le gène *pol*. L'ARN rétroviral comprend également au moins deux gènes additionnels qui sont les gènes *gag* et *env*. Le gène *gag* code pour les protéines du squelette, c'est à dire pour la matrice, la capside et la nucléocapside. Le gène env code pour les protéines d'enveloppe. La transcription est régulée par des régions promotrices situées au niveau des LTR (Long Terminal Repeat) encadrant les extrémités 5' et 3' terminales du génome rétroviral.

Au cours de l'évolution les humains ou leurs ancêtres ont intégré dans leur génome du matériel d'origine rétrovirale suite à une infection. En effet, lors de l'infection d'une cellule, la reverse transcriptase fait une copie ADN de l'ARN rétroviral, cette copie d'ADN pouvant alors éventuellement s'intégrer dans le génome humain. Les rétrovirus peuvent infecter les cellules germinales et se transmettre ainsi aux générations futures par transmission Mendélienne verticale. On parle alors de rétrovirus endogènes qui sont présents sous la forme d'ADN proviral intégré dans le génome de toutes les cellules humaines. La plupart des rétrovirus endogènes sont silencieux ou défectifs. Toutefois certains d'entre eux ont pu conserver tout ou partie de leurs propriétés initiales et peuvent être activés dans des conditions particulières. L'expression des rétrovirus endogènes peut aller de la transcription de gènes viraux jusqu'à la production de particules virales.

Ces rétrovirus endogènes peuvent être associés directement ou indirectement au développement de certaines pathologies.

Des structures rétrovirales endogènes peuvent se retrouver sous une forme complète LTR-gag-pol-env-LTR ou sous des formes tronquées.

Ainsi, dans une précédente demande de brevet (PCT/FR98/01442), la Demanderesse a criblé une banque d'ADNc à l'aide d'une sonde Ppol-MSRV (SEQ ID NO:18) et détecté des clones chevauchants qui lui ont permis de reconstruire un ARN génomique putatif de 7582 nucléotides. Cet ARN génomique présente une structure R-U5-gag-pol-env-U3-R. Une interrogation « blastn » sur plusieurs bases de données à l'aide du génome reconstruit a permis de montrer qu'il existe une quantité importante de séquences génomiques (ADN) apparentées dans le génome humain qui sont trouvées sur plusieurs chromosomes. Ainsi, la Demanderesse a mis en évidence l'existence de structures partielles de type rétroviral dans le génome humain et envisagé leur rôle potentiel dans le développement de maladies auto-immunes, dans des insuccès de grossesse ou des états de grossesse pathologiques.

A titre d'exemple on peut citer comme maladie auto-immune la sclérose en plaques, la polyarthrite rhumatoïde, le lupus érythémateux disséminé, le diabète insulino-dépendant et/ou les pathologies qui leur sont associées.

L'isolement et le séquençage de fragments d'ADNc chevauchants et l'identification de clones génomiques (ADN) correspondant aux clones d'ADN isolés, décrits dans la demande de brevet PCT précitée de la Demanderesse sont incorporés ici à titre de référence.

Isolement et séquençage de fragments d'ADNc chevauchants :
Les informations concernant l'organisation de la nouvelle famille de rérovirus endogènes dénommée par la Demanderesse HERV-W ont été obtenues en testant une banque d'ADNc placentaire (Clontech cat#HL5014a) avec les sondes Ppol-MSRV (SEQ ID NO:18) et Penv-C15 (SEQ ID NO:19) et en pratiquant ensuite une technique de "gene walking" à l'aide des nouvelles séquences obtenues. Les expériences ont été mises en oeuvre en se référant aux préconisations du fournisseur de la banque. Des amplifications PCR sur ADN ont également été exploitées pour comprendre cette organisation.

Les clones suivants ont été sélectionnés et séquencés :
- Clone c1.6A2 (SEQ ID NO:20) : région 5' non traduite de HERV-W et une partie de gag
- Clone c1.6A1 (SEQ ID NO:21) : gag et une partie de pol
- Clone cl.7A16 (SEQ ID NO:22): Région 3' de pol
- Clone cl.Pi22 (SEQ ID NO:23) : région 3' de pol et début de env
- Clone c1.24.4 (SEQ ID NO:24) : ARN épissé comprenant une partie de la région 5' non traduite de HERV-W, la fin de pol et la région 5' de env
- Clone cl.C4C5. (SEQ ID NO:25) : fin de env et région 3' non traduite de HERV-W
- Clone cl.PH74 (SEQ ID NO:26) : ARN sous-génomique : région 5' non traduite de HERV-W, fin de pol, env, et région 3' non traduite de HERV-W
- Clone cl.PH7 (SEQ ID NO:27) : ARN multi-épissé : région 5' non traduite de HERV-W, fin de env et région 3' non traduite de HERV-W.
- Clone cl.Pi5T (SEQ ID NO:28) : gène pol partiel et région U3-R
- Clone c1.44.4 (SEQ ID NO:29) : région R-U5, gène gag et gène pol partiel.

A l'aide de ces clones, en procédant à des alignements de séquences, un modèle de séquence totale de HERV-W a été élaboré. Les ARN épissés ont été mis en évidence ainsi que les sites potentiels donneurs et accepteurs d'épissage. Par étude de similitude avec des rétrovirus existants, les entités LTR, gag, pol et env ont été définies.

L'organisation génétique putative de HERV-W sous forme ARN est la suivante (SEQ ID NO:30) :
gène 1..7582

Localisation des clones sur la séquence ARN génomique reconstruite
cl.6A2 (1321 pb) 1-1325 ;
cl.PH74 (535+2229= 2764 pb) 72-606 et 5353-7582;
cl.24.4 (491+1457= 1948 pb); 115-606 et 5353-6810;
cl.44.4 (2372 pb) 115-2496 ;
cl.PH7 (369+297= 666pb) 237-606 et 7017-7313; c1.6A1 (2938 pb) 586-3559.;
cl.Pi5T (2785+566= 3351 pb) 2747-5557 et 7017-7582;
c1.7A16 (1422 pb) 2908-4337;
cl.Pi22 (317+1689 = 2006 pb) 3957-4273 et 4476-6168;
c1.C4C5 (1116 pb) 6467-7582

| | |
|---|---|
| 5'LTR | 1..120 |
| | /note="R of 5'LTR (extrémité 5' |
| | incertaine" |
| | 121..575 |
| | /note="U5 of 5'LTR" |
| divers | 579..596 |
| | /note="PBS primer binding site pour tRNA-W" |
| divers | 606 |
| | /note="jonction d'épissage (site donneur d'épissage ATCCAAAGTG-GTGAGTAATA et site accepteur d'épissage CTTTTTTCAG-ATGGGAAACG clone RG083M05, GenBank accession AC000064)" |
| divers | 5353 |
| | /note="site accepteur d'épissage pour l'ORF1 (env)" |
| divers | 5560 |
| | /note="site donneur d'épissage" |
| ORF | 5581..7194 |
| | /note="ORF1 env 538 AA" /produit-="enveloppe" |
| divers | 7017 |
| | /note="site accepteur d'épissage pour ORF2 et ORF3" |
| ORF | 7039..7194 |
| | /note="ORF2 52 AA" |
| ORF | 7112..7255 |
| | /note="ORF3 48 AA" |
| divers | 7244..7254 |
| | /note="PPT polypurine tract" |
| 3'LTR | 7256..7582 |
| | /note-="U3-R of 3' LTR (jonction U3-R indéterminée) |
| divers | 7563..7569 |
| | signal de polyadénylation |

Identification de clones génomiques (ADN) correspondant aux clones d'ADN isolés :
Une interrogation "blastn" sur plusieurs bases de données, à l'aide du génome reconstruit, a montré qu'il existe une quantité importante de séquences apparentées dans le génome humain. Environ 400 séquences ont été identifiées dans GenBank et plus de 200 séquences dans la banque EST, la plupart en anti-sens. Les 4 séquences les plus significatives en taille et en similitude sont les séquences des clones génomiques (ADN) suivants :
   le clone humain RG083M05 (gb AC000064) dont la localisation chromosomique est 7q21-7q22,
   le clone humain BAC378 (gb U85196, gb AE000660) correspondant au locus alpha delta du récepteur des cellules T, localisé en 14q11-12,
   le cosmide humain Q11M15 (gb: AF045450) correspondant à la région 21q22.3 du chromosome 21,
   le cosmide U134E6 (embl Z83850) sur le chromosome Xq22.

La localisation des régions alignées pour chacun des clones est indiquée et l'appartenance à un chromosome est indiquée entre crochets (figure 6). Le pourcentage de similitude (sans les larges délétions) entre les 4 séquences et l'ARN génomique reconstruit est indiqué, ainsi que la présence de séquences répétées à chaque extrémité du génome et la taille des plus grandes trames de lecture (ORF). Des séquences répétées ont été trouvées aux extrémités de 3 de ces clones. La séquence reconstruite est contenue intégralement à l'intérieur du clone RG083M05 (9, 6 Kb) et présente une similitude de 96%. Cependant le clone RG083M05 présente une insertion de 2 Fb située immédiatement en aval de la région 5' non traduite (5' UTR). Cette insertion est également trouvée dans deux autres clones génomiques qui présentent une délétion de 2, 3 Kb immédiatement en amont de la région 3' non traduite (3' UTR). Aucun clone ne contenait les trois trames de lecture (ORFs) gag, pol et env fonctionnelles. Le clone RG083M05 montre une ORF de 538 acides aminés (AA) correspondant à une enveloppe entière. Le cosmide Q11M15 contient deux grandes ORFs contigues de 413 AA (trame 0) et 305 AA (trame +1) correspondant a une polyprotéine pol tronquée.

On a maintenant trouvé et isolé un fragment nucléique endogène, intégré à l'ADN du génome humain, qui comprend ou consiste en au moins une partie du gène *gag* d'un rétrovirus endogène associé à une maladie auto-immune, ou à des insuccès de grossesse ou pathologies de la grossesse, cette partie au moins codant, directement ou indirectement, pour un produit d'expression.

Avantageusement, le fragment précédemment défini répond en outre à au moins l'une quelconque des caractéristiques suivantes :
il comprend, ou consiste en, ledit gène *gag* entier ;
ladite partie du fragment au moins code pour la matrice et la capside ;
il comprend, ou consiste en, SEQ ID NO:1, SEQ ID NO:2, SEQ ID NO:3, ou le complémentaire de l'une quelconque desdites séquences ;
il est localisé sur au moins l'un des chromosomes humain 1, 3, 6, 7 et 16, de préférence il est localisé au moins sur le chromosome 3 ;
le produit d'expression de ladite partie est de l'ARN messager ;
le produit d'expression de ladite partie est immunologiquement reconnu par des anticorps présents dans un échantillon biologique d'un patient atteint d'une maladie auto-immune, telle que la sclérose en plaques ; de préférence, le fluide biologique est choisi parmi le sèrum, le plasma, le liquide synovial et l'urine.

Il est aussi décrit un produit de transcription endogène, à l'état isolé, susceptible d'être obtenu par transcription d'au moins ladite partie du gènes *gag* d'un fragment précité.

L'invention concerne aussi un procédé de détection d'un fragment nucléique endogène appartenant au gène gag du rétrovirus humain endogène HERV-W, dans un échantillon rétrovirus humain endogène HERV-W, dans un échantillon biologique, comprenant les étapes suivantes :
on effectue au préalable une étape d'extraction de l'ADN cellulaire dudit échantillon, on réalise au moins un cycle d'amplification de l'ADN cellulaire, telle que par PCR, au moyen d'amorces choisies parmi SEQ ID NO:4 à SEQ ID NO:9 et SEQ ID NO:12 à SEQ ID NO : 17,
on effectue une étape de transcription / traduction in vitro du produit amplifié,
on fait réagir le produit issu de l'étape de transcrption / traduction avec un sérum ou plasma d'un patient présentant la solérose en plaques, et
on détecte au moins un produit d'expression choisi parmi un ARN messager dont la traduction conduit à un polypeptide consistant en SEQ ID NO : 31, et un polypeptide consistant en SEQ ID NO : 31.

L' invention concerne encore un procédé pour l'étude et/ou le suivi d'une prolifération cellulaire suivi d'une prolifération cellulaire des cellules T in vitro selon lequel on met en contact les cellules T d'un patient avec soit des produits de transcription / tradiction (SEQ ID NO : 31), tels qu'obtenus selon le procédé ci-dessus, soit des peptides de synthèse dérivés de ou appartenant à SEQ ID NO : 31.

Avantageusement, la sonde est, marquée par un traceur, tel que par exemple un traceur radioactif ou une enzyme.

Le fragment precité trouve les applications suivantes :
Une protéine recombinante obtenue à partir d'une cassette d'expression dans un hôte bactérien caractérisée en ce que à séquence protéique consiste en SEQ ID NO :31 ; en particulier l'hôte bactérien est *E. coli*.
Un réactif pour la détection d'une maladie auto-immune ou un suivi de grossesse comprenant au moins un fragment ou une protéine décrit précédemment ;
L'utilisation d'un fragment ou d'une protéine décrit précédemment pour détecter, dans un échantillon biologique, une susceptibilité à une maladie auto-immune ou un suivi de grossesse ; en particulier, La maladie auto-immune est la sclérose en plaques.

Avant d'exposer la présente invention plus en détail, la définition de certains termes employés dans la description et les revendications: est donnée.

L'expression « produit d'expression » signifie tout produit dérivé de l'ADN rétroviral intégré dans le génome humain incluant les produits de la transcription (ARN messager) et les produits issus de la traduction de l'ARN messager obtenu. Dans ce dernier cas, et à titre d'exemple, le produit peut être un peptide, une protéine fonctionnelle ou fonctionnalisable, c'est-à-dire susceptible de devenir fonctionnelle.

Par partie codant, directement ou indirectement, pour un produit d'expression, on entend une partie qui à elle seule comprend au moins tout ou partie d'un cadre de lecture ouvert duquel on peut déduire une séquence d'acides aminés, et dont la capacité codante peut être induite par des éléments tels que par exemple ceux susceptibles d'avoir une activité promotrice. Cette définition inclut la variabilité qui peut être retrouvée dans la séquence nucléique codante, sous réserve que les conditions ci-dessus soient respectées.

### Exemple 1: Localisation du gène gag de la famille HERV-W sur les chromosomes humains par la technique de Southern blot

Afin de localiser le gène gag de la famille HERV-W, une sonde correspondante à ce gène de MSRV a été hybridée sur une membrane de Nylon (Hybond^{®} N+, Amersham) contenant 5 µg d'ADN de 24 hybrides somatique de cellules [humain x rongeurs] (de l'ADN génomique humain isolé: 22 chromosomes autosomes et 2 chromosomes sexuels) et 3 ADN contrôles (humain, souris et hamster) digérés par l'enzyme de restriction EcoRI.

La sonde utilisée est la suivante : Pgag-Cl2 identifiée par SEQ ID NO:3 correspondant à la région codante (de 1056 pb) du clone MSRV gag Cl2.

### 1.1- Obtention du clone 2, C12, contenant en 3'une partie homologue au gène pol, correspondant au gène protéase, et une partie homologue au gène gag correspondant à la nucléocapside et une région 5' codante, correspondant au gène gag, plus spécifiquement la matrice et la capside de MSRV-1.

Une amplification par PCR a été effectuée sur de l'ARN total extrait à partir de 100 µl de plasma d'un patient atteint de SEP. Un témoin eau, traité dans les mêmes conditions, a été utilisé comme contrôle négatif. La synthèse de cDNA a été réalisée avec 300 pmoles d'une amorce aléatoire (Gibco-BRL, France) et la transcriptase inverse « Expand RT » (Boehringer Mannheim, France) selon les conditions préconisées par la société. Une amplification par PCR (polymerase chain reaction) a été effectuée avec l'enzyme *Taq* polymerase (Perkin Elmer, France) en utilisant 10 µl de cDNA dans les conditions suivantes : 94°C 2 min, 55°C 1 min, 72°C 2 min puis 94°C 1 min, 55°C 1 min, 72°C 2 min pendant 30 cycles et 72°C pendant 7 min et avec un volume réactionnel final de 50 µl.

Les amorces utilisées pour l'amplification par PCR sont les suivantes :
- amorce 5', identifiée par SEQ ID NO:4
   5' CGG ACA TCC AAA GTG ATG GGA AAC G 3' ;
- amorce 3', identifiée par SEQ ID NO:5
   5' GGA CAG GAA AGT AAG ACT GAG AAG GC 3'

Une deuxième amplification par PCR dite « nichée » a été réalisée avec des amorces 3' et 5' situées à l'intérieur de la région déjà amplifiée. Cette deuxième PCR a été effectuée dans les mêmes conditions expérimentales que celles utilisées lors de la première PCR, en utilisant 10 µl du produit d'amplification issu de la première PCR.

Les amorces utilisées pour l'amplification par PCR nichée sont les suivantes :
- amorce 5', identifiée par SEQ ID NO:6
   5' CCT AGA ACG TAT TCT GGA GAA TTG GG 3' ;
- amorce 3', identifiée par SEQ ID NO:7
   5' TGG CTC TCA ATG GTC AAA CAT ACC CG 3'

Un produit d'amplification de 1511 pb a été obtenu à partir de l'ARN extrait du plasma de patient SEP. Le fragment correspondant n'a pas été observé pour le témoin eau. Ce produit d'amplification a été cloné de la façon suivante.

L'ADN amplifié a été inséré dans un plasmide à l'aide du kit TA Cloning". Les 2 µl de solution d'ADN ont été mélangés avec 5 µl d'eau distillée stérile, 1 µl d'un tampon de ligation 10 fois concentré « 10X Ligation Buffer », 2 µl de « PCR^{®} Vector » (25 ng/ml) et 1 µl de « T4 DNA Ligase ». Ce mélange a été incubé une nuit à 14°C. Les étapes suivantes ont été réalisées conformément au instructions du kit TA cloning^{®} (Invitrogen). Le mélange de ligation a été étalé après transformation de la ligation dans des bactéries *E*. *coli*. A la fin de la procédure, les colonies blanches de bactéries recombinantes ont été repiquées pour être cultivées et permettre l'extraction des plasmides incorporés selon la procédure dite de « minipréparation d'ADN » (J. Sambrook, E.F . Fritsch and T. Maniatis, Molecular Cloning, a laboratory manual, Cold Spring Harbour Laboratory Press, 1989). La préparation de plasmide de chaque colonie recombinante a été coupée par l'enzyme de restriction Eco RI et analysée sur gel d'agarose. Les plasmides possédant un insert détecté sous lumière UV après marquage du gel au bromure d'éthidium, ont été sélectionnés pour le séquençage de l'insert, après hybridation avec une amorce complémentaire du promoteur T7 présent sur le plasmide de clonage du TA cloning kit^{®}. La réaction préalable au séquençage a ensuite été effectuée selon la méthode préconisée pour l'utilisation du kit de séquençage « Prism^{®} Ready Réaction Amplitaq^{®} FS, DyeDeoxy^{™} Terminator » (Applied Biosystems, ref. 402119) et le séquençage automatique a été réalisé sur les appareils 373 A et 377 Applied Biosystems, selon les instructions du fabricant.

Le clone obtenu, dénommé C12, permet de définir une région de 1511 pb présentant une phase ouverte de lecture dans la région N-terminale de 1089 pb (fragment 434-1521 de SEQ ID NO:2) codante pour 363 acides aminés (SEQ ID NO:31) correspondant aux régions matrice et capside du gène *gag.*

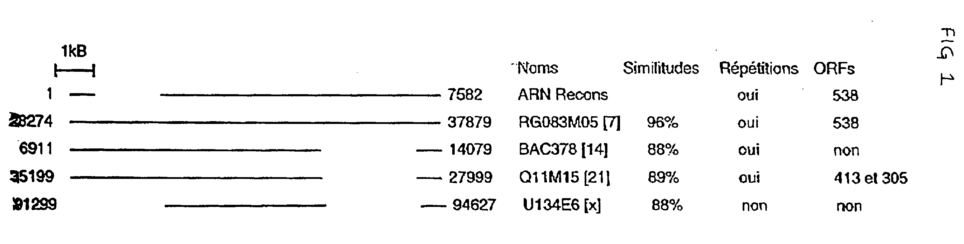

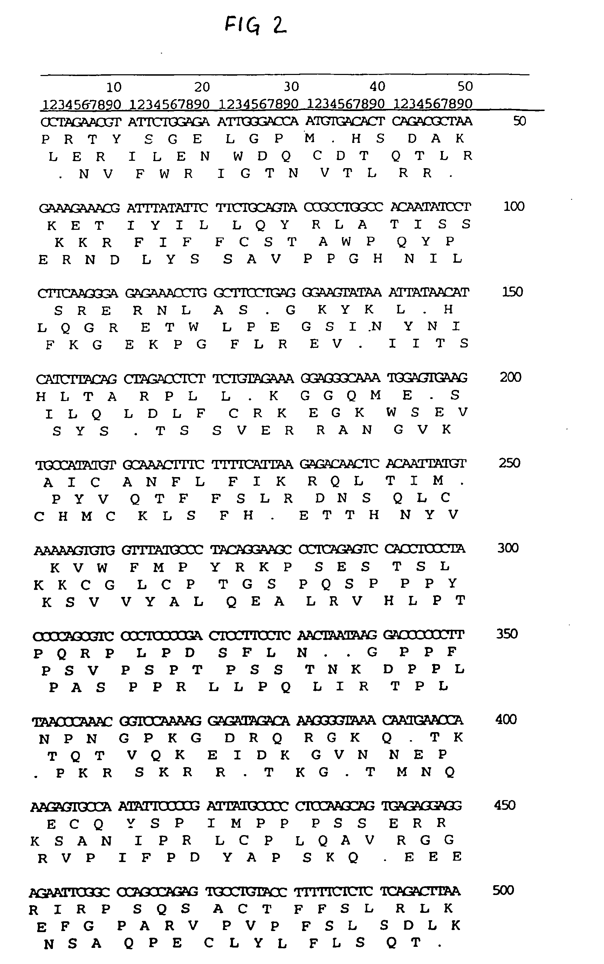

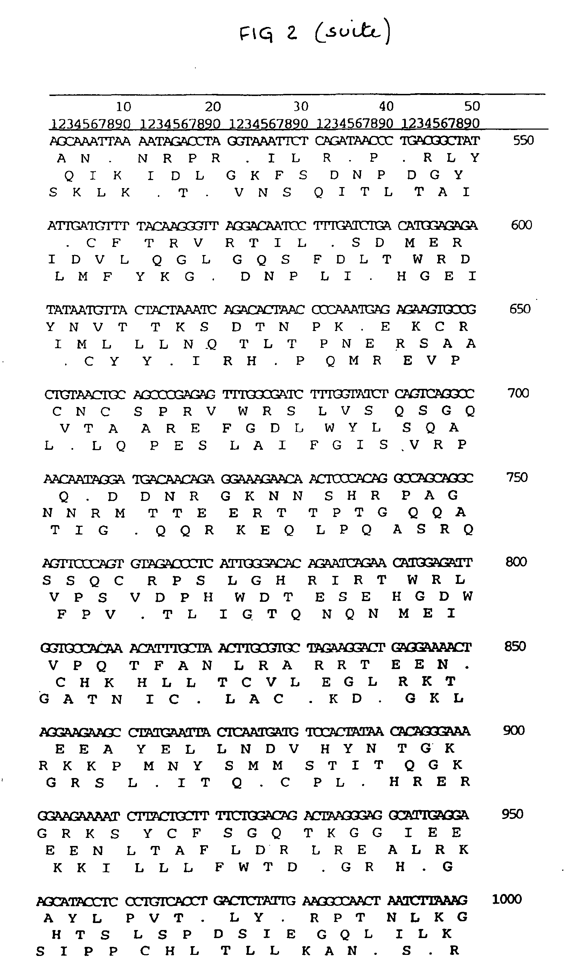

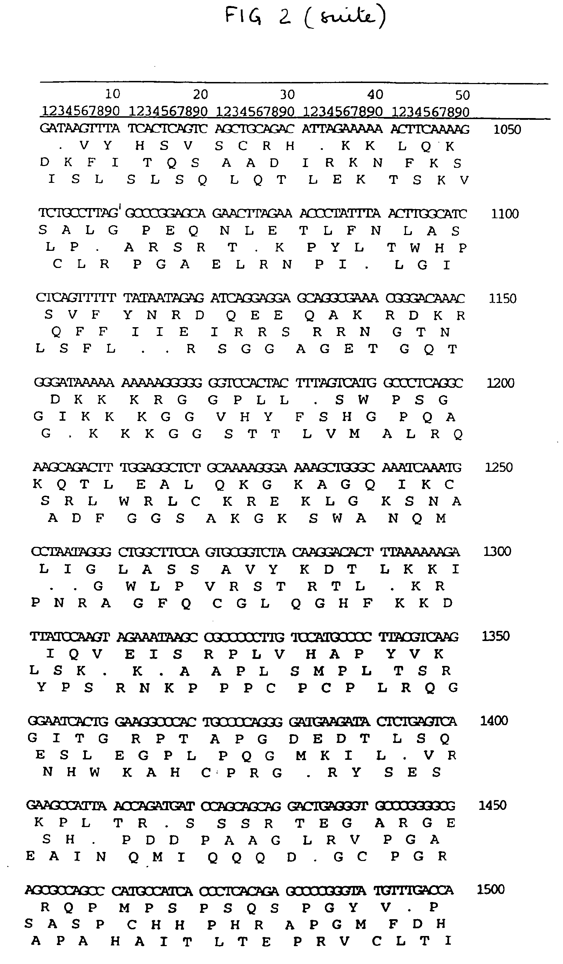

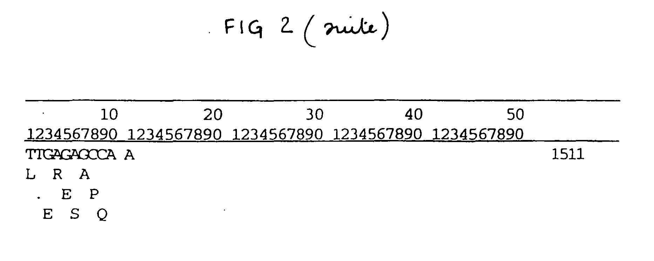

La séquence nucléotidique de C12 est identifiée par SEQ ID NO:1. Elle est représentée à la figure 2 avec les trames de lecture potentielles en aminoacides.

### 1.2- obtention de la sonde gag cl2 MSRV

La sonde a été obtenue après amplification par PCR, à partir du plasmide pCR^{™} vector (kit TA Cloning^{®}, Invitrogen) contenant l'insert du clone: gag c12 de MSRV, avec la *Taq* polymérase (Perkin Elmer, France) dans les conditions suivantes : 94°C 1 min, 55°C 1 min, 72°C 2 min pendant 35 cycles et 72°C pendant 7 min et avec un volume réactionnel final de 100 µl.

Les amorces utilisées pour l'amplification par PCR sont les suivantes :
- amorce 5', identifiée par SEQ ID NO:12
   5'-CTA GAA CGT ATT CTG GAG AAT TGG GA-3'
- amorce 3', identifiée par SEQ ID NO:13
   5'-CCT AAG GCA GAC TTT TGA AG -3'

Un produit d'amplification de 1056 pb a été obtenu pour gag cl2 de MSRV.

Après l'amplification par PCR, le fragment a été analysé en gel d'agarose 1%. Le fragment détecté sous lumière UV, après marquage du gel, au bromure d'éthidium, a été coupé et marqué au [α-P³²] à l'aide des amorces aléatoires (Gibco-BRL, France) conformément aux instructions du kit « Ready to go DNA labelling » (Pharmacia Biotech). Les nucléotides non incorporés ont été éliminés avec une colonne G-50 Quick Spin (Boehringer, Mannheim).

### 1.3- Southern blot

Les conditions d'hybridation sont les suivantes :
Après 4 heures de pré-hybridation (en 5x SSC, 1X Denhardt's, 0,1% SDS, 50% formamide, 20 mM Tris-HCl pH = 7,5, 0,1 mg/ml d'ADN de sperme de hareng), la membrane de Nylon contenant les chromosomes humains a été hybridée (en 5x SSC, 1x Denhardt's, 0,1% SDS, 50% formamide, 20 mM Tris-HCl pH = 7,5, 0,1 mg/ml d'ADN de sperme de hareng, 5% sulfate de dextran) pendant 18 heures à 42°C, avec la sonde d'ADN de 1056 pb (SEQ ID NO:3) de gag cl2 marquée au ³²P. Après hybridation, la membrane (The BIOS Monochromosomal Somatic Cell Hybrid blot, de Quantum Bioprobe) hybridée avec la sonde gag a été lavée en solution 2x SSC, 0,2% SDS 2 fois 15 min, à température ambiante, et (en 0,2x SSC = 0,2% SDS) 2 fois 15 min, à 45°C. Après lavage, la membrane a été exposée au film aux rayons X, à -80°C en présence d'écran amplificateur.

Les résultats sont présentés dans le tableau 1 ci-après.

Dans ce tableau :
m signifie souris (mouse) et h signifie hamster et correspondent aux cellules receveuses de l'ADN de chromosomes humains.
le chiffre indiqué sous chaque chromosome correspond au nombre de bandes rencontrées.

Le nombre total de copies du gène gag est de 66.

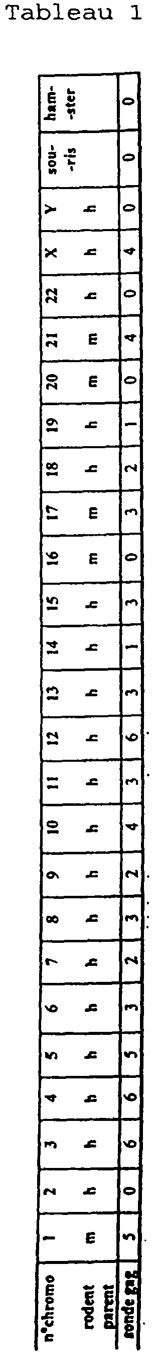

### Exemple 2: Amplification par PCR du gène gag de la famille HERV-W sur chacun des chromosomes humains isolés ; vérification de la spécificité des amplifications par Southern blot ; test de transcription-traduction « in vitro » (PTT) à partir des produits de PCR, afin de vérifier la capacité codante, repérer quels sont les chromosomes humains présentant des trames de lecture ouvertes pour le gène gag de la famille HERV-W.

### 2.1- Amplification par PCR

Pour l'amplification du gène *gag* HERV-W, une PCR a été effectuée sur chaque chromosome humain isolé [*NIGMS human*/*rodent somatic cell hybrid panel #2.* The human monochromosomal NIGMS somatic hybrid mapping panel #2, décrit par H.L. Drwinga et all et B.L. Dubois et al) obtenu de Coriell Institute (Camden, NJ)] avec l'enzyme *Taq* polymérase (Perkin Elmer, France) en utilisant: 40 pmol de chaque amorce, 25 mM de chaque dNTP (Pharmacia), 2,5 mM MgCl₂, 2,5 U de *Taq* polymérase dans le tampon PCR standard (Perkin Elmer), et 300 ng DNA de chromosome isolé dans un volume final de 100 µl. Les conditions de PCR pour amplifier la région *gag* sont les suivantes : 3 min à 94°C; puis 1 min à 94°C, 1 min à 55°C, 3 min à 72°C, pendant 30 cycles et 7 min à 72°C.

Les amorces utilisées pour l'amplification, par PCR, du gène *gag* à partir d'un ATG introduit dans la séquence gag HERV-W sur chaque chromosome humain isolé sont les suivantes :
- amorce 5', identifiée par SEQ ID NO:14

L'amorce contient la séquence du promoteur T7 RNA polymérase, un « spacer », la séquence Kozak (site d'initiation à la traduction chez les eucaryotes) et la séquence 5' gag à partir de l'ATG de HERV-W.
- amorce 3', identifiée par SEQ ID NO:15
   5'-TTTTTTTTTTTTTTTTTTTCAGGCTGCGCCAGTGTCCAGGAGAC-3'

L'amorce contient une queue de poly-A (pour stabiliser la transcription de l'ARN, représentée par 18 bases T), un codon stop (représenté par TCA) et la séquence du gène protéase (G+E+A) de MSRV-1.

Pour l'amplification du gène gag HERV-W en utilisant des oligonucléotides définis dans les régions LTR et protéase de HERV-W avec l'enzyme *Taq* polymérase (Perkin Elmer, France), les conditions de PCR ont été les suivantes : 3 min à 94°C; puis 1 min à 94°C, 1 min à 60°C, 2 min à 72°C, 35 cycles ; suivi de 7 min à 72°C, avec 50 ng de chaque ADN monochromosomal.

Les amorces utilisées pour l'amplification, par PCR, du gène *gag* à l'aide de l'oligonucléotide défini dans la séquence LTR HERV-W sur chaque chromosome humain isolé, sont les suivantes :
- amorce 5', identifiée par SEQ ID NO:16
   5'-TGTCCGCTGTGCTCCTGATC-3'
- amorce 3', identifiée par SEQ ID NO:17
   5'-TTTTTTTTTTTTTTTTTTTCAGGCTGCGCCAGTGTCCAGGAGAC-3'

L'amorce contient une queue de poly-A (pour stabiliser la transcription de l'ARN, représenté par 18 bases T), un codon stop (représenté par TCA) et la séquence du gène protéase de G+E+A de MSRV-1.

Les amplification par PCR ont été effectuées dans l'appareil MJ Research PTC200, Peltier Thermal cycler. Les produits de PCR (10 µl de chaque produit PCR) ont été analysés dans un gel 1% agarose en TBE 1X(Tris-HCl, borate, EDTA). Afin de vérifier la spécificité des produits d'amplification, 3 µl de chaque produit PCR ont été analysés en gel d'agarose et puis transférés sur une membrane de Nylon (Hybond^{®}-N⁺, Amersham) (Southern blot) à l'aide de NaOH 0,4N. L'hybridation avec la sonde gag cl2 (1056 pb) (J. Sambrook et al, 1989) a été effectuée dans les conditions suivantes : après 4 heures de pré-hybridation (en 5x SSC, 1X Denhardt's, 0,1% SDS, 50% formamide, 20 mM Tris-HCl pH = 7,5, 0,1 mg/ml d'ADN de sperme de hareng), la membrane de Nylon a été hybridée (en 5x SSC, 1x Denhardt's, 0,1% SDS, 50% formamide, 20 mMTris-HCl pH = 7,5, 0,1 mg/ml d'ADN de sperme de hareng, 5% sulfate de dextran) pendant 18 heurs à 42°C, avec la sonde d'ADN gag marquée au ³²P. Les produits de PCR gag de chaque chromosome humain isolé ont été lavés en solution de 2x SSC, 0,2% SDS 1 fois, 15 min à température ambiante; 0,2x SSC, 0,1% SDS 2 fois 15 min chaque lavage à 65°C, en 0,1x SSC, 0,1% SDS 2 fois 15 min chaque à 65°C, et en 0,1x SSC, 0,1% SDS 2 fois 30 min chaque à température ambiante.

Une partie du volume restant (4 µl) des produits d'amplification par PCR a été utilisée pour le test de transcription-traduction « in vitro » PTT, (Roest PAM et al, 1993) (Promega, France). Le volume restant a été utilisé pour le clonage dans le vecteur pCR^{®} 2.1-TOPO (Invitrogen) conformément aux instructions du kit, et pour le séquençage avec la méthode préconisée pour l'utilisation du kit de séquençage « PRISM^{™} Ready Reaction Amplitaq^{®} FS, DyeDeoxy^{™} Terminator » (Applied Biosystems, réf. 402119) et le séquençage automatique a été réalisé sur les appareils 373A et 377 Applied Biosystems, selon les instructions du fabricant.

La partie codée (SEQ ID NO:31) par le fragment de 2009 pb (SEQ ID NO:2) a été amplifié par PCR avec l'enzyme *Pwo* (5U/µl) (Boehringer Mannheim, France) en utilisant 1 µl de la mini-préparation de l'ADN du clone gag (SEQ ID NO :3) sous les conditions suivantes : 95°C 1 min, 60°C 1 min, 72°C 2 min pendant 25 cycles et avec un volume réactionnel final de 50 µl à l'aide des amorces:
- amorce 5' (*Bam* HI) (SEQ ID NO :8):
   5' ATG GGA AAC GTT CCC CCC GAG 3' (21 mers), et
- amorce 3' (*Hind* III), identifiée par SEQ ID NO:9
   5 GGC CTA AGG CAG ACT TTT GAA 3' (21 mers)

Le fragment obtenu après PCR, a été linéarisé par *Bam* HI et *Hind* III et sous-cloné dans les vecteurs d'expression pET28C et pET21C (NOVAGEN) linéarisé par *Bam* HI et *Hind* III. Le séquençage de l'ADN du fragment de 1089 pb dans les deux vecteurs d'expression a été réalisé selon la méthode préconisée pour l'utilisation du kit de séquençage "PRISMT^{™} Ready Reaction Amplitaq^{®} FS, DyeDeoxy^{™} Terminator" (Applied Biosystems, ref. 402119) et le séquençage automatique a été réalisé sur les appareils 373 A et 377 Applied Biosystems, selon les instructions du fabricant.

L'expression de la séquence nucléotidique du fragment de 1089 pb du clone gag par les vecteurs d'expression pET28C et pET21C sont identifiées par respectivement SEQ ID NO:10 et SEQ ID NO:11.

### 2.2- Test de transcription-traduction « in vitro » (PTT, Promega)

Ce test a été effectué pour repérer les chromosomes humains présentant des trames de lecture ouvertes pour le gène *gag* de la famille HERV-W.

Dans un volume réactionnel de 25 µl, un mélange contenant 12,5 µl de lysat de réticulocyte de lapin TNT^{®} (Promega), 1 µl de tampon de réaction TNT^{®} (Promega), 0,5 µl d'ARN polymérase TNT^{®} (Promega), 0,5 µl de mélange à 1mM des acides aminés moins méthionine, 2 µl de ³⁵S-méthionine (1.000 Ci/mmol) à 10 mCi/µl (Amersham), 0,5 µl d'inhibiteur de ribonucléase RNasin^{®} à 40 U/µl, 4 µl de produits d'amplification par PCR (équivalent à 1 µg) de chaque chromosome humain et 4 µl d'eau. Ce mélange a été incubé à 30°C pendant 90 min.

Les protéines gag correspondant aux produits de transcription/traduction du gène gag de la famille HERV-W de chaque chromosome humain, amplifié par PCR, ont été mises en évidence par électrophorèse sur gel de polyacrylamide à 10% en présence de Dodécyl Sulfate de Sodium (SDS)-PAGE après exposition du gel au film aux rayons X à température ambiante en présence d'écran amplificateur.

Les résultats sont présentés dans le tableau 2 ci-après.

Dans ce tableau, le chiffre indiqué sous chaque chromosome correspond à la masse moléculaire (kDa) des protéines visualisées en gel de polyacrylamide en présence de SDS.

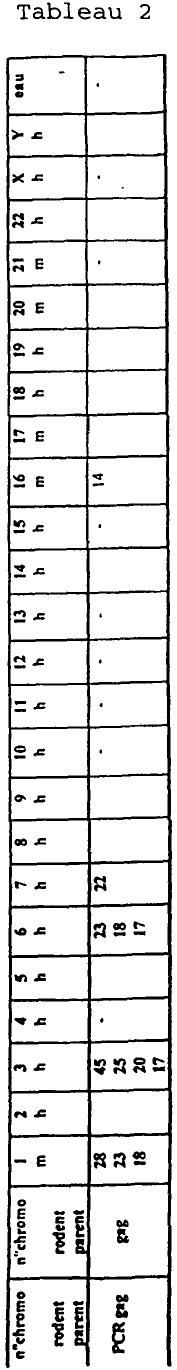

### Exemple 3 : expression du clone gag chez Escherichia coli, et réaction avec des sérums humains

On a exprimé la partie codante SEQ ID NO:2 chez *Escherichia coli* puis on a testé les produits ainsi exprimés vis à vis de sérum de patients atteints de SEP, ainsi que de sérum de patients sains.

Les constructions pET28c-clone gag (1089 pb) et pET21C-clone gag (1089 pb) synthétisent, dans la souche bactérienne BL21 (DE3), une protéine en fusion N- et C-terminale pour le vecteur pET28C et C-Terminale pour le vecteur pET21C avec 6 résidus histidine, de masse moléculaire apparente d'environ 45 kDa, mise en évidence par électrophorèse sur gel de polyacrylamide SDS-PAGE (U.K. Laemmli, Cleavage of structural proteins during the assembly of the head of bacteriophage T4, Nature, 1970, 227 :680-685).

La réactivité de la protéine a été mise en évidence vis à vis d'un anticorps monoclonal anti-Histidine (DIANOVA) par la technique de Western blot (H. Towbin et al., Electrophoretic transfer of proteins from polyacrylamid gels to nitrocellulose skeets : procedure and some applications, Proc. Natl. Acad. Sci. USA., 1979, 76 :4350-4354).

Les protéines recombinantes pET28c-clone gag (1089 pb) et pET21C-clone gag (1089 pb) ont été visualisées en SDS-PAGE dans la fraction insoluble après digestion enzymatique des extraits bactériens avec 50 µl de lysozyme (10 mg/ml) et lyse par ultrasons.

Les propriétés antigéniques des antigènes recombinants pET28C-clone gag (1089 pb) et pET21C-clone gag (1089 pb) ont été testées par Western Blot après solubilisation du culot bactérien avec 2% SDS et 50 mM β-mercaptoéthanol. Après incubation avec les sérums de patients atteints de sclérose en plaques, les sérums des témoins neurologiques et les sérums de témoins de centre de transfusion sanguine (CTS), les immunocomplexes ont été détectés à l'aide d'un sérum de chèvre anti-IgG et anti-IgM humaines, couplé à la phosphatase alcaline.

Les résultats sont présentés dans le tableau 3 ci-après.

**Tableau 3**

| Réactivité de sérums de patients atteints de sclérose en plaques et témoins avec la protéine recombinante de gag produite chez *E. coli^{a}* | | |
|---|---|---|
| MALADIE | NOMBRE D'INDIVIDUS TESTÉS | NOMBRE D'INDIVIDUS POSITIFS |
| SEP | 15 | 6 2 (+++), 2 (++), 2 (+) |
| TEMOINS NEUROLOGIQUES | 2 | 1(+++) |
| TEMOINS SAINS (CTS) | 22 | 1(+/-) |

(a) Les bandelettes contenant 1,5 µg d'antigène recombinant gag présentent une réactivité contre de sérums dilués au 1/100. L'interprétation de Western Blot est basée sur la présence ou absence d'une bande gag spécifique sur les bandelettes. Des contrôles positifs et négatifs sont inclus dans chaque expérience.

Ces résultats montrent que, dans les conditions techniques utilisées, environ 40% des sérums humains atteints de sclérose en plaques testés réagissent avec la protéine recombinante gag.

## Revendications

1. Procédé de détection d'un fragment nucléique endogène appartenant au gène gag du rétrovirus humain endogène HERV-W, dans un échantillon biologique, comprenant les étapes suivantes :
- on extrait l'ADN cellulaire dudit échantillon,
- on amplifie l'ADN extrait avec une sonde choisie parmi SEQ ID NOs : 4 à 9 et 12 à 17,
- on effectue une étape de transcription/traduction in vitro du produit amplifié,
- on fait réagir le produit issu de l'étape de transcription/traduction avec un sérum ou un plasma d'un patient présentant la sclérose en plaques, et
- on détecte au moins un produit d'expression choisi parmi un ARN messager dont la traduction conduit à un polypeptide consistant en SEQ ID NO: 31 et un polypeptide consistant en SEQ ID NO : 31.

## Claims

1. A method for detecting an endogenous nucleic fragment belonging to the gag gene of the endogenous human retrovirus HERV-W, in a biological sample, comprising the following steps:
- the cell DNA is extracted from said sample,
- the extracted DNA is amplified with a probe selected from SEQ ID NOs: 4 to 9, and 12 to 17,
- an *in vitro* transcription/translation step of the amplified product is carried out,
- the product from the transcription/translation step is reacted with a serum or a plasma of a patient having multiple sclerosis, and
- at least one expression product selected from a messenger RNA, the translation of which leads to a polypeptide consisting in SEQ ID NO: 31, and a polypeptide consisting in SEQ ID NO: 31 are detected.

## Patentansprüche

1. Verfahren zur Erkennung eines endogenen Nukleinsäurefragments, das zum gag-Gen des endogenen humanen Retrovirus HERV-W gehört, in einer biologischen Probe, das die folgenden Schritte umfasst:
- Extraktion der zellulären DNA aus der Probe,
- Amplifizierung der extrahierten DNA mit einer Sonde, ausgewählt aus den SEQ ID-Nr. 4 bis 9 und 12 bis 17,
- Durchführung eines Schritts der in vitro-Transkription / Translation des amplifizierten Produkts,
- Versetzen in Reaktion des Produkts nach dem Schritt der Transkription / Translation gewonnenen Produkts mit einem Serum oder einem Plasma eines Patienten, der die Multiple Sklerose aufweist, und
- Detektion mindestens eines Expressionsprodukts, ausgewählt aus einer Messenger-RNA, dessen Translation zu einen Polypeptid führt, das aus SEQ ID Nr. 31 besteht, und einem Polypeptid, das aus SEQ ID Nr. 31 besteht.


### LISTE DE SEQUENCES

<110> BIO MERIEUX
<120> Fragment nucléique endogène associé à une maladie auto-immune, procédé de marquage et réactif
<130> SEP19-endogène
<140>
   <141>
<150> FR 9900888
   <151> 1999-01-21
<160> 31
<170> PatentIn Ver. 2.1
<210> 1
   <211> 1511
   <212> ADN
   <213> Homo sapiens
<400> 1
   cctagaacgt attctggaga attgggacca atgtgacact cagacgctaa gaaagaaacg 60
   atttatattc ttctgcagta ccgcctggcc acaatatcct cttcaaggga gagaaacctg 120
   gcttcctgag ggaagtataa attataacat catcttacag ctagacctct tctgtagaaa 180
   ggagggcaaa tggagtgaag tgccatatgt gcaaactttc ttttcattaa gagacaactc 240
   acaattatgt aaaaagtgtg gtttatgccc tacaggaagc cctcagagtc cacctcccta 300
   ccccagcgtc ccctccccga ctccttcctc aactaataag gacccccctt taacccaaac 360
   ggtccaaaag gagatagaca aaggggtaaa caatgaacca aagagtgcca atattccccg 420
   attatgcccc ctccaagcag tgagaggagg agaattcggc ccagccagag tgcctgtacc 480
   tttttctctc tcagacttaa agcaaattaa aatagaccta ggtaaattct cagataaccc 540
   tgacggctat attgatgttt tacaagggtt aggacaatcc tttgatctga catggagaga 600
   tataatgtta ctactaaatc agacactaac cccaaatgag agaagtgccg ctgtaactgc 660
   agcccgagag tttggcgatc tttggtatct cagtcaggcc aacaatagga tgacaacaga 720
   ggaaagaaca actcccacag gccagcaggc agttcccagt gtagaccctc attgggacac 780
   agaatcagaa catggagatt ggtgccacaa acatttgcta acttgcgtgc tagaaggact 840
   gaggaaaact aggaagaagc ctatgaatta ctcaatgatg tccactataa cacagggaaa 900
   ggaagaaaat cttactgctt ttctggacag actaagggag gcattgagga agcatacctc 960
   cctgtcacct gactctattg aaggccaact aatcttaaag gataagttta tcactcagtc 1020
   agctgcagac attagaaaaa acttcaaaag tctgccttag gcccggagca gaacttagaa 1080
   accctattta acttggcatc ctcagttttt tataatagag atcaggagga gcaggcgaaa 1140
   cgggacaaac gggataaaaa aaaaaggggg ggtccactac tttagtcatg gccctcaggc 1200
   aagcagactt tggaggctct gcaaaaggga aaagctgggc aaatcaaatg cctaataggg 1260
   ctggcttcca gtgcggtcta caaggacact ttaaaaaaga ttatccaagt agaaataagc 1320
   cgcccccttg tccatgcccc ttacgtcaag ggaatcactg gaaggcccac tgccccaggg 1380
   gatgaagata ctctgagtca gaagccatta accagatgat ccagcagcag gactgagggt 1440
   gcccggggcg agcgccagcc catgccatca ccctcacaga gccccgggta tgtttgacca 1500
   ttgagagcca a                                                      1511
<210> 2
   <211> 2009
   <212> ADN
   <213> Homo sapiens
<400> 2
   atacgactac tatagggcga attgggccct ctagatgcat gctcgagcgg ccgccagtgt 60
   gatggatatc tgcagaattc gccctttgtc cgctgtgctc ctgatccagc gaggcgccca 120
   ttgctgctcc caattgggct aaaggcttgc cattgttccc acacggctaa gtgcccgggt 180
   tcatcctaat tgagctgaac actagtcact gggttccatg gttctcttcc atgacccacg 240
   gcttctaata gagctctaat actcaccaca tggcccaaga ttccattcct tggaatccgt 300
   gaggccaaga accccaggtc agagaacacg aggcttgcca ccgtcttgga agtggcccgc 360
   cgccatcttg ggagctctgg gagcaaggac cccccagtaa cattttggca accacaaagg 420
   gacctccaaa gcgatgggaa acattccccc caaggcaaaa acgcccctaa gatgtattct 480
   ggagaattgg gaccaatgtg acactcagac gctaagaaag aaacgattta tattcttctg 540
   cagtaccgcc tggccacaat atcctcttca agggagagaa acctggcttc ctgagggaag 600
   tataaattat aacatcatct tacagctaga cctcttctgt agaaaggagg gcaaatggag 660
   tgaagtgcca tatgtgcaaa ctttcttttc attaagagac aactcacaat tatgtaaaaa 720
   gtgtggttta tgccctacag gaagccctca gagtccacct ccctacccca gcgtcccccc 780
   cccgactcct tcctcaacta ataaggaccc ccctttaacc caaacggtcc aaaaggagat 840
   agacaaaggg gtaaacaatg aaccaaagag tgccaatatt ccccgattat gccccctcca 900
   agcagtgaga ggaggagaat tcggcccagc cagagtgcct gtaccttttt ctctctcaga 960
   cttaaagcaa attaaaatag acctaggtaa attctcagat aaccctgacg gctatattga 1020
   tgttttacaa gggttaggac aatcctttga tctgacatgg agagatataa tgttactact 1080
   aaatcagaca ctaaccccaa atgagagaag tgccgctgta actgcagccc gagagtttgg 1140
   cgatctttgg tatctcagtc aggtcaacaa taggatgaca acagaggaaa gaacaactcc 1200
   cacaggccag caggcagttc ccagtgtaga ccctcattgg gacacagaat cagaacatgg 1260
   agattggtgc cacaaacatt tgctaacttg cgtgctagaa ggactgagga aaactaggaa 1320
   gaagcctatg aattactcaa tgatgtccac tataacacag ggaaaggaag aaaatcctac 1380
   tgcttttctg gacagactaa gggaggcatt gaggaagcat acctccctgt cacctgactc 1440
   tattgaaggc caactaatct taaaggataa gtttatcact cagtcagctg cagacattag 1500
   aaaaaacttc aaaagtctgc cttaggcccg gagcagaact tagaaaccct atttaacttg 1560
   gcatcctcag ttttttataa tagagatcag gaggagcagg cgaaacggga caaacgggat 1620
   aaaaaaaaaa gggggggtcc actactttag tcatggccct caggcaagca gactttggag 1680
   gctctggaaa agggaaaagc tgggcaaatc aaatgcctaa tagggctggc ttccagtgcg 1740
   gtctacaagg acactttaaa aaagattatc caagtagaaa taagccgccc ccttgtccat 1800
   gccccttacg tcaagggaat cactggaagg cccactgccc caggggatga agatactctg 1860
   agtcagaagc cattaaccag atgatccagc agcaggactg agggtgcccg gggcgagcgc 1920
   cagcccatgc catcaccctn acagagcccc gggtatgctt gaccattgag agccaggagg 1980
   ttaactgtct cctggacact ggcgcagcc 2009
<210> 3
   <211> 1056
   <212> ADN
   <213> Homo sapiens
<400> 3
   ctagaacgta ttctggagaa ttgggaccaa tgtgacactc agacgctaag aaagaaacga 60
   tttatattct tctgcagtac cgcctggcca caatatcctc ttcaagggag agaaacctgg 120
   cttcctgagg gaagtataaa ttataacatc atcttacagc tagacctctt ctgtagaaag 180
   gagggcaaat ggagtgaagt gccatatgtg caaactttct tttcattaag agacaactca 240
   caattatgta aaaagtgtgg tttatgccct acaggaagcc ctcagagtcc acctccctac 300
   cccagcgtcc cctccccgac tccttcctca actaataagg accccccttt aacccaaacg 360
   gtccaaaagg agatagacaa aggggtaaac aatgaaccaa agagtgccaa tattccccga 420
   ttatgccccc tccaagcagt gagaggagga gaattcggcc cagccagagt gcctgtacct 480
   ttttctctct cagacttaaa gcaaattaaa atagacctag gtaaattctc agataaccct 540
   gacggctata ttgatgtttt acaagggtta ggacaatcct ttgatctgac atggagagat 600
   ataatgttac tactaaatca gacactaacc ccaaatgaga gaagtgccgc tgtaactgca 660
   gcccgagagt ttggcgatct ttggtatctc agtcaggcca acaataggat gacaacagag 720
   gaaagaacaa ctcccacagg ccagcaggca gttcccagtg tagaccctca ttgggacaca 780
   gaatcagaac atggagattg gtgccacaaa catttgctaa cttgcgtgct agaaggactg 840
   aggaaaacta ggaagaagcc tatgaattac tcaatgatgt ccactataac acagggaaag 900
   gaagaaaatc ttactgcttt tctggacaga ctaagggagg cattgaggaa gcatacctcc 960
   ctgtcacctg actctattga aggccaacta atcttaaagg ataagtttat cactcagtca 1020
   gctgcagaca ttagaaaaaa cttcaaaagt ctgcct                           1056
<210> 4
   <211> 25
   <212> ADN
   <213> Homo sapiens
<400> 4
   cggacatcca aagtgatggg aaacg 25
<210> 5
   <211> 26
   <212> ADN
   <213> Homo sapiens
<400> 5
   ggacaggaaa gtaagactga gaaggc 26
<210> 6
   <211> 26
   <212> ADN
   <213> Homo sapiens
<400> 6
   cctagaacgt attctggaga attggg 26
<210> 7
   <211> 26
   <212> ADN
   <213> Homo sapiens
<400> 7
   tggctctcaa tggtcaaaca tacccg 26
<210> 8
   <211> 21
   <212> ADN
   <213> Homo sapiens
<400> 8
   atgggaaacg ttccccccga g 21
<210> 9
   <211> 21
   <212> ADN
   <213> Homo sapiens
<400> 9
   ggcctaaggc agacttttga a 21
<210> 10
   <211> 409
   <212> PRT
   <213> Homo sapiens
<400> 10
<210> 11
   <211> 393
   <212> PRT
   <213> Homo sapiens
<400> 11
   Met Ala Ser Met Thr Gly Gly Gln Gln Met Gly Arg Ile Met Gly Asn
   1 5 10 15
   Ile Pro Pro Lys Ala Lys Thr Pro Leu Arg Cys Ile Leu Glu Arg Ile
   20 25 30
   Leu Glu Asn Trp Asp Gln Cys Asp Thr Gln Thr Leu Arg Lys Lys Arg
   35 40 45
   Phe Ile Phe Phe Cys Ser Thr Ala Trp Pro Gln Tyr Pro Leu Gln Gly
   50 55 60
   Arg Glu Thr Trp Leu Pro Glu Gly Ser Ile Asn Tyr Asn Ile Ile Leu
   65 70 75 80
   Gln Leu Asp Leu Phe Cys Arg Lys Glu Gly Lys Trp Ser Glu Val Pro
   85 90 95
   Tyr Val Gln Thr Phe Phe Ser Leu Arg Asp Asn Ser Gln Leu Cys Lys
   100 105 110
   Lys Cys Gly Leu Cys Pro Thr Gly Ser Pro Gln Ser Pro Pro Pro Tyr
   115 120 125
   Pro Ser Val Pro Ser Pro Thr Pro Ser Ser Thr Asn Lys Asp Pro Pro
       130                 135                 140
   Leu Thr Gln Thr Val Gln Lys Glu Ile Asp Lys Gly Val Asn Asn Glu
   145                 150                 155                 160
   Pro Lys Ser Ala Asn Ile Pro Arg Leu Cys Pro Leu Gln Ala Val Arg
                   165                 170                 175
   Gly Gly Glu Phe Gly Pro Ala Arg Val Pro Val Pro Phe Ser Leu Ser
               180                 185                 190
   Asp Leu Lys Gln Ile Lys Ile Asp Leu Gly Lys Phe Ser Asp Asn Pro
           195                 200                 205
   Asp Gly Tyr Ile Asp Val Leu Gln Gly Leu Gly Gln Ser Phe Asp Leu
       210                 215                 220
   Thr Trp Arg Asp Ile Met Leu Leu Leu Asn Gln Thr Leu Thr Pro Asn
   225                 230                 235                 240
   Glu Arg Ser Ala Ala Val Thr Ala Ala Arg Glu Phe Gly Asp Leu Trp
                   245                 250                 255
   Tyr Leu Ser Gln Ala Asn Asn Arg Met Thr Thr Glu Glu Arg Thr Thr
               260                 265                 270
   Pro Thr Gly Gln Gln Ala Val Pro Ser Val Asp Pro His Trp Asp Thr
           275                 280                 285
   Glu Ser Glu His Gly Asp Trp Cys His Lys His Leu Leu Thr Cys Val
       290                 295                 300
   Leu Glu Gly Leu Arg Lys Thr Arg Lys Lys Pro Met Asn Tyr Ser Met
   305                 310                 315                 320
   Met Ser Thr Ile Thr Gln Gly Lys Glu Glu Asn Leu Thr Ala Phe Leu
                   325                 330                 335
   Asp Arg Leu Arg Glu Ala Leu Arg Lys His Thr Ser Leu Ser Pro Asp
               340                 345                 350
   Ser Ile Glu Gly Gln Leu Ile Leu Lys Asp Lys Phe Ile Thr Gln Ser
           355                 360                 365
   Ala Ala Asp Ile Arg Lys Asn Phe Lys Ser Leu Pro Lys Leu Ala Ala
       370                 375                 380
   Ala Leu Glu His His His His His His
   385                 390
<210> 12
   <211> 26
   <212> ADN
   <213> Homo sapiens
<400> 12
   ctagaacgta ttctggagaa ttggga 26
<210> 13
   <211> 20
   <212> ADN
   <213> Homo sapiens
<400> 13
   cctaaggcag acttttgaag 20
<210> 14
   <211> 54
   <212> ADN
   <213> Homo sapiens
<400> 14
   tttggtaata cgactcacta tagggcagcc accatgggaa acgttccccc cgag 54
<210> 15
   <211> 44
   <212> ADN
   <213> Homo sapiens
<400> 15
   tttttttttt tttttttttc aggctgcgcc agtgtccagg agac 44
<210> 16
   <211> 20
   <212> ADN
   <213> Homo sapiens
<400> 16
   tgtccgctgt gctcctgatc 20
<210> 17
   <211> 44
   <212> ADN
   <213> Homo sapiens
<400> 17
   tttttttttt tttttttttc aggctgcgcc agtgtccagg agac 44
<210> 18
   <211> 678
   <212> ADN
   <213> Homo sapiens
<400> 18
   tcagggatag cccccatcta tttggccagg cattagccca agacttgagc cagttctcat 60
   acctggatat tcttgtcctt tggtatgcgg atgatttact tttagccgcc cgttcagaaa 120
   ccttgtgcca tcaagccacc caagtgctct taaatttcct cgccacctgt ggctacaagg 180
   tttccaaacc aaaggctcag ctctgctcac agcagaaggc tatttaccct aaatacttag 240
   ggctgaaatt atccaaaggc accagggccc tcagtgagga atgtatccag cctatactgg 300
   cttatcctta tcccaaaacc ctaaaacaac taagaaggtt ccttggcata ataggcataa 360
   caggcataac aggtttctgc tgaatatgga ttcccaagta cggcaaaata gccagaccat 420
   tatatacact aattaaggaa actcagaaag ccaataccca tttagtaaga tggacacctg 480
   aagcagaggc agctttccag gccgtaaaga acaccctaac ccaagcccca gtgttaagct 540
   tgccagcggg gcaagacttt tctttctgtg tcacagaaaa aataggaata gctntaggag 600
   tccttacaca ggtccgaggg accagcttgc aacccatggc atacctgagt aaggaaattg 660
   atgtagtggc aaagggtt 678
<210> 19
   <211> 591
   <212> ADN
   <213> Homo sapiens
<400> 19
   ccatggccat ctacactgaa caagatttat acaatcatgt cgtacctaag ccccacaaca 60
   aaagagtacc cattcttcct tttgttatca gagcaggagt gctaggcaga ctaggtactg 120
   gcattggcag tatcacaacc tctactcagt tctactacaa actatctcaa gaaataaatg 180
   gtgacatgga acaggtcact gactccctgg tcaccttgca agatcaactt aactccctag 240
   cagcagtagt ccttcaaaat cgaagagctt tagacttgct aaccgccaaa agagggggaa 300
   cctgtttatt tttaggagaa gaacgctgtt attatgttaa tcaatccaga attgtcactg 360
   agaaagttaa agaaattcga gatcgaatac aatgtagagc agaggagctt caaaacaccg 420
   aacgctgggg cctcctcagc caatggatgc cctgggttct ccccttctta ggacctctag 480
   cagctctaat attgttactc ctctttggac cctgtatctt taacctcctt gttaagtttg 540
   tctcttccag aattgaagct gtaaagctac agatggtctt acaaatctag a 591
<210> 20
   <211> 1321
   <212> ADN
   <213> Homo sapiens
<400> 20
   caacaatcgg gatataaacc caggcattcg agctggcaac agcagccccc ctttgggtcc 60
   cttccctttg tatgggagct gttttcatgc tatttcactc tattaaatct tgcaactgca 120
   ctcttctggt ccatgtttct tacggctcga gctgagcttt tgctcaccgt ccaccactgc 180
   tgtttgccac caccgcagac ctgccgctga ctcccatccc tctggatcct gcagggtgtc 240
   cgctgtgctc ctgatccagc gaagcgccca ttgccgctcc caattgggct aaaggcttgc 300
   cattgttcct gcacggctaa gtgcctgggt ttgttctaat tgagctgaac actagtcact 360
   gggttccatg gttctcttct gtgacccacg gcttctaata gaactataac acttaccaca 420
   tggcccaaga ttccattcct tggaatccgt gaggccaaga actccaggtc agagaatacg 480
   aagcttgcca ccatcttgga agcggcctgc taccatcttg gaagtggttc accaccatct 540
   tgggagctct gtgagcaagg accccccggt aacattttgg caaccacgaa cggacatcca 600
   aagtgatggg aaacgttccc cgcaagacaa aaacgcccct aagacgtatt ctggaaaatt 660
   gggaacaatt tgaccctcag acactaagaa agaaacgact tatattcttc tgcagtgccg 720
   cctggcactc ctgagggaag tataaattat aacaccatct tacagctaga cctcttttgt 780
   agaaaaggca aatggagtga agtgccataa gtacaaactt tcttttcatt aagagacaac 840
   tcacaattat gtaaaaagtg tgatttatgc cctacaggaa gccttcagag tctacctccc 900
   tatcccagca tccccgactc cttccccact taataaggac cccccttcaa cccaaatggt 960
   ccaaaaggag atagacaaaa gggtaaacag tgaaccaaag agtgccaata ttccccaatt 1020
   atgacccctc caagcagtgg gaggaagaga attcggccca gccagagtgc atgtgccttt 1080
   ttctctccca gacttaaagc aaataaaaac agacttaggt aaattctcag ataaccctga 1140
   tggctatatt ggtgttttac aagggttagg acaattcttt gatctgacat ggagagatat 1200
   atatgtcact gctaaatcag acactaaccc caaatgagag aagtgccacc ataactgcag 1260
   cctgagagtt tggcgatctc tggtatctca gtcaggtcaa tgataggatg acaacagagg 1320
   a 1321
<210> 21
   <211> 2938
   <212> ADN
   <213> Homo sapiens
<400> 21
   caacgacgga catccaaagt gatgggaaac gttccccgca agacaaaaac gcccctaaga 60
   cgtattctgg agaattggga ccaatttgac cctcagacac taagaaagaa acgacttata 120
   ttcttctgca gtgccgcctg gcactcctga gggaagtata aattataaca ccatcttaca 180
   gctagacttc ttttgtagaa aaggcaaatg gagtgaagtg ccataagtac aaactttctt 240
   ttcattaaga gacaactcac aattatgtaa aaagtgtgat ttatgcccta caggaagcct 300
   tcagagtcta cctccctatc ccagcatccc cgactccttc cccaactaat aaggaccccc 360
   cttcaaccca aatggtccaa aaggagatag acaaaagggt aaacagtgaa ccaaagagtg 420
   ccaatattcc ccaattatga cccctcccaa gcagtgggag gaagagattc ggcccagcca 480
   gagtgcatgt gctttttctt ctcccagact taaagcaaat aaaaacagac ttaggtaaat 540
   tctcagataa tcctgatggc tatattgatg ttttacaagg gttaggacaa ttctttgatc 600
   tgacatggag agatataatg tcactgctaa atcagacact aaccccaaat gagagaagtg 660
   ccaccataac tgcagcctga gagtttggcg atctctggta tctcagtcag gtcaatgata 720
   ggatgacaac agaggaaaga gatgatcccc acagccagca agcagttccc agtctasacc 780
   ctcattgggg acacagaaat cagtaacatg ggagattggt gctgcagaca tttgctaact 840
   tgtgtgctac aaggactaag gaaaactacg aagaaaatct acgaattact caatgatgtc 900
   caccataaca caggggaagg gaagaaaatc ctactgcctt tctggagaga ctaagggagg 960
   cattgaggaa gcgtgcctct ctgtcacctg actcttctga aggccaacta atcttaaagc 1020
   gtaagtttat cactcagtca gctgcagaca ttagaaaaaa cttcaaaagt ctgccgtagg 1080
   cccggagcaa aacttagaaa ccctattgaa cttggcaacy tcggtttttt ataatagaga 1140
   tcaggaggag caggcggaac aggacaaacg ggattaaaaa aaaggccacc gctttagtca 1200
   tgaccctcag gcaagtggac tttggaggct ctggaaaagg gaaaagctgg gcaaattgaa 1260
   tgcctaatag ggcttgcttc cagtgcggtc tacaaggaca ctttaaaaaa gattgtccaa 1320
   gtagaagtaa gccgcccctt cgtccatgcc ccttatttca agggaatcac tggaaggccc 1380
   actgccccag gggacaaagg tcttttgagt cagaagccac taaccagatg atccagcagc 1440
   aggactgagg gtgcctgggg caagcgccat cccatgccat caccctcaca gagccctggg 1500
   tatgcttgac cattgagggc caggaaggtt gtctcctgga cactggtgcg gtcttcttag 1560
   tcttactctt ctgtcccgga caactgtcct ccagatctgt cactatctga gggggtccta 1620
   agacgggcag tcactagata cttctcccag ccactaagtt atgactgggg agctttattc 1680
   ttttcacatg cttttctaat tatgcttgaa agccccacta ccttgttagg gagagacatt 1740
   ctagcaaaag caggggccat tatacacctg aacataggag aaggaacacc cgtttgttgt 1800
   cccctgcttg aggaaggaat taatcctgaa gtctgggcaa cagaaggaca atatggacga 1860
   gcaaagaatg cccgtcctgt tcaagttaaa ctaaaggatt ccacttcctt tccctaccaa 1920
   aggcagtacc ccctcagacc caaggcccaa caaggattcc aaaagattgt taaggactta 1980
   aaagcccaag gcttagtaaa accatgcata actccctgca gtaattccgt agtggattga 2040
   ggaggcacag aaacccagtg gacagtggag ggttagtgca agatctcagg attatcaatg 2100
   gaggccgttg tccttttata cccagctgta cctagccctt atactgtgct ttcccaaata 2160
   ccagaggaag cagagtggtt tacactcctg gaccttaagg atgccttctt ctgcatccct 2220
   gtacatcctg actctcaatt cttgtttgcc tttgaagata cttcaaaccc aacatctcaa 2280
   ctcacctgga ctgttttacc ccaagggttc agggatagcc cccatctatt tggccaggca 2340
   ttagcccaag acttgagcca atcctcatac ctggacactt gtccttcggt aggtggatga 2400
   tttacttttg gccgcccatt cagaaacctt gtgccatcaa gccacccaag cgctcttcaa 2460
   tttcctcgct acctgtggct acatggtttc caaaccaaag gctcaactct gctcacagca 2520
   ggttacttag ggctaaaatt atccaaaggc accagggccc tcagtgagga acacatccag 2580
   cctatactgg cttatcctca tcccaaaacc ctaaagcaac taaggggatt ccttggcgta 2640
   ataggtttct gccgaaaatg gattcccagg tttggcgaaa tagccaggtc attaaataca 2700
   ctaattaagg aaactcagaa agccaatacc catttagtaa gatggacaac tgaagtagaa 2760
   gtggctttcc aggccctaac ccaagcccca gtgttaagtt tgccaacagg gcaagacttt 2820
   tcttcatatg tcacagaaaa aacaggaata gctctaggag tccttacaca gatccgaggg 2880
   atgagcttgc aacctgtggc gtacctgact aaggaaattg atgtagtggc aaagggtt 2938
<210> 22
   <211> 1422
   <212> ADN
   <213> Homo sapiens
<400> 22
   tcagggatag cccccatcta tttggccagg cattagccca agacttgagt cagttatcat 60
   acctggacac tcttgtcctt cagtatgtgg atgatttact tttagctgcc tgttcagaaa 120
   ccttgtgcca tcaagccacc caagcactct taaatttcct cgccacctgt ggctacaagg 180
   tttccaaaga gaagctcagc tctgctcaca gcaggttaaa tacttaggac taagattatc 240
   caaaggcacc aaggccctca gtgaggaatg tatccagcct atactggctt atcctcatct 300
   caaaacccta aagcaactaa gagagttcct tggcataaca ggcttctgcc gaatatggat 360
   tccccaggta tggcaaaata gccaggccat tatatacagt aattaaggaa actcagaaag 420
   ccaataccca tttaataaga tggatacctg aagccaaagt ggctttccag gcccctaaag 480
   aaggccttaa acccaagtcc cagtgttaag cttgccaacg gggcaagact tttctttata 540
   catcacagaa aaaaacagaa acagctctgg gagtccttac acaggtccaa gggacgagct 600
   tgcaacccat ggcatacctg agtaaggaaa ctgatgtagt ggcaaagggt tggcttcatt 660
   gtttatgggt agtggtggca gtagcagttg tagtatctga agcagttaaa ataatacagg 720
   ggagagatct tactgtgtgg acatctcatg aggtgaacag catactcact gctaaaggag 780
   acttgtggct gtcagacaac cgtttactta aatatcaggc tctattactt gaaaggccag 840
   tgctgcaact gtgcacttgt gcaactctta acccagtcnc atttcttcca gacaatgaag 900
   atagaatata actgtcaaca aataatttct caaacctatg ccactcgagg ggaccttcta 960
   gaagttccct tgactgatcc tgaccttcaa cttgtatact gatggaagtt cctttgtaga 1020
   aaaaggactt caaaagcggg gtatgcagtg gtcagtgata atggaatatt tgaaagtatc 1080
   ccctcactcc aggaactagt gcttagctgg cagaactaat agccttcatt ggggcactag 1140
   aattaggaga aggaaaaagg gtaaatatat atacagactc tgagtatgct cacctagtcn 1200
   tccatgccca tgaggcaata tgcagagaaa gggaattcct aacttccgag ggaacaccta 1260
   tcacacatca ggaagccatt aggagattat tactggcagt acagaaacct aaagaggtgg 1320
   aagtcttaca ctgctggggt catcagaaag gaaagaaaag ggaaatagaa gggaattgcc 1380
   aagcagatat tgaagcaaaa agagctgcaa ggcaggaccc tc                    1422
<210> 23
   <211> 2006
   <212> ADN
   <213> Homo sapiens
<400> 23
   atgcagtggt cagtgataat ggaatacttg aaagtaatcc cctcactcca ggaactagtg 60
   ctcagctagc agaactaata gccctcactt gggcactaga attaggagaa gaaaaaaggg 120
   caaatatata tacagactct aaatatgctt acctagtcct ccatgcccat gcagcaatat 180
   ggaaagaaag ggaattccta acttctgaga gaacacctat caaacatcag gaagccatta 240
   ggaaattatt attggctgta cagaaaccta aagaggtggc agtcttacac tgccggggtc 300
   atcanaaagg aaaggaaagg gaaaatactt ttgcctgcaa ctatccaatg gaaattactt 360
   aaaacccttc atcaaacctt tcacttaggc atcgatagca cccatcaaat ggccaaatca 420
   ttatttactg gaccaggcct tttcaaaact atcaagcaaa tattcagggc ctgtgaattg 480
   tgccaaaaaa ataatcccct gcctcatcgc caagctcctt caggaaaaca aaaaacaggc 540
   cattaccctg aaaaaaactg gcaactgatt ttacccacaa gcccaaacct cagggatttc 600
   agtatctact agtctgggta aatactttca cgggttgggc aaaggccttc ccctgtagga 660
   cagaaaaggc ccaagaggta ataaaggcac tagttcatga aataattccc agattcggac 720
   ttccccgagg cttacagagt gacaatagcc ctgctttcca ggccacagta acccagggag 780
   tatcccaggc gttaggtata cgatatcact tacactgcgc ctgaaggcca cagtcctcag 840
   ggaaggtcga gaaaatgaat gaaatactca aaggacatct aaaaaagcaa acccaggaaa 900
   cccacctcac atggcctgct ctgttgccta tagccttaaa aagaatctgc aactttcccc 960
   aaaaagcagg acttagccca tacgaaatgc tgtatggaag gcccttcata accaatgacc 1020
   ttgtgcttga cccaagacag ccaacttagt tgcagacatc acctccttag ccaaatatca 1080
   acaagttctt aaaacattac aaggaaccta tccctgagaa gagggaaaag aactattcca 1140
   cccttgtgac atggtattag tcaagtccct tctctctaat tccccatccc tagatacatc 1200
   ctgggaagga ccctacccag tcattttatt taccccaact gcggttaaag tggctggagt 1260
   ggtcttggat acatcacact tgagtcaaat cctggatact gccaaaggaa cctgaaaatc 1320
   caggagacaa cgctagctat tcctgtgaac ctctagagga tttgcgcctg ctcttcaaac 1380
   aacaaccagg aggaaagtaa ctaaaatcat aaatccccca tggccctccc ttatcatatt 1440
   tttctcttta ctgttctttt accctctttc actctcactg caccccctcc atgccgctgt 1500
   atgaccagta gctcccctta ccaagagttt ctatggagaa tgcagcgtcc cggaaatatt 1560
   gatgccccat cgtataggag tctttctaag ggaaccccca ccttcactgc ccacacccat 1620
   atgccccgca actgctatca ctctgccact ctttgcatgc atgcaaatac tcattattgg 1680
   acaggaaaaa tgattaatcc tagttgtcct ggaggacttg gagtcactgt ctgttggact 1740
   tacttcaccc aaactggtat gtctgatggg ggtggagttc aagatcaggc aagagaaaaa 1800
   catgtaaaag aagtaatctc ccaactcacc cgggtacatg gcacctctag ccctacaaag 1860
   gactagatct ctcaaaacta catgaaaccc tccgtaccca tactcgcctg gtaagcctat 1920
   ttaataccac cctcactggg ctccatgagg tctcggccca aaaccctact aactgttgga 1980
   tatgcctccc cctgaacttc aagcca                                      2006
<210> 24
   <211> 1948
   <212> ADN
   <213> Homo sapiens
<400> 24
   actgcactct tctggtccat gtttcttacg gctcgagctg agcttttgct caccgtccac 60
   cactgctgtt tgccaccacc gcanacctgc cgctgactcc catccctctg gatcctgcag 120
   ggtgtccgct gtgctcctga tccagcgagg cgcccattgc cgctcccaat tgggctaaag 180
   gcttgccatt gtncctgcac ggctaagtgc ctgggtttgt tctaattgag ctgaacacta 240
   ntcactgggt tccatggttc tcttctgtga cccacggctt ctaatagaac tataacactt 300
   accacatggc ccaagattcc attccttgga atccgtgagg gcaagaactc caggtcagag 360
   aatacgaggc ttgccaccat cttggaagcg gcctgctacc atcttggaag tggttcacca 420
   ccatcttggg agctctgtga gcaaggaccc cccggtaaca ttttggcaac cacgaacgga 480
   catccaaagt gatacatcct gggaaggacc ctacccagtc attttatcta ccccaactgc 540
   ggttaaagtg gctggagtgg agtcttggat acatcacact tgagtcaaat cctggatact 600
   gccaaaggaa cctgaaaatc caggagacaa cgctagctat tcctgtgaac ctctagagga 660
   tttgcgcctg ctcttcaaac aacaaccagg aggaaagtaa ctaaaatcat aaatccccat 720
   ggccctccct tatcatattt ttctctttac tgttgtttca ccctctttca ctctcactgc 780
   accccctcca tgccgctgta tgaccagtag ctccccttac caagagtttc tatggagaat 840
   gcagcgtccc ggaaatattg atgccccatc gtataggagt ctttgtaagg gaacccccac 900
   cttcactgcc cacacccata tgccccgcaa ctgctatcac tctgccactc tttgcatgca 960
   tgcaaatact cattattgga caggaaaaat gattaatcct agttgtcctg gaggacttgg 1020
   agtcactgtc tgttggactt acttcaccca aactggtatg tctgatgggg gtggagttca 1080
   agatcaggca agagaaaaac atgtaaaaga agtaatctcc caactcaccc gggtacatgg 1140
   cacctctagc cctacaaagg gactagatct ctcaaaacta catgaaaccc tccgtaccca 1200
   tactcgcctg gtaagcctat ttaataccac cctcactggg ctccatgagg tctcggccca 1260
   aaaccctact aactgttgga tatgcctccc cctgaacttc aggccatatg tttcaatccc 1320
   tgtacctgaa caatggaaca acttcagcac agaaataaac accacttccg ttttagtagg 1380
   acctcttgtt tccaatctgg aaataaccca tacctcaaac ctcacctgtg taaaatttag 1440
   caatactaca tacacaacca actcccaatg catcaggtgg gtaactcctc ccacacaaat 1500
   agtctgccta ccctcaggaa tattttttgt ctgtggtacc tcagcctatc gttgtttgaa 1560
   tggctcttca gaatctatgt gcttcctctc attcttagtg ccccctatgg ccatctacac 1620
   tgaacaagat ttatacagtt atgtcatatc taagccccgc aacaaaagag tacccattct 1680
   tccttttgtt ataggagcag gagtgctagg tgcactaggt actggcattg gcggtatcac 1740
   aacctctact cagttctact acaaactatc tcaagaacta aatggggaca tggaacgggt 1800
   cgccgactcc ctggtcacct tgcaagatca acttaactcc ctagcagcag tagtccttca 1860
   aaatcgaaga gctttagact tgctaaccgc tgaaagaggg ggaacctgtt tatttttagg 1920
   ggaagaatgc tgttattatg ttaatcaa                                    1948
<210> 25
   <211> 1136
   <212> ADN
   <213> Homo sapiens
<400> 25
   ccatggccat ctacactgaa caagatttat acagttatgt catatctaag ccccgcaaca 60
   aaagagtacc cattcttcct tttgttatag gagcaggagt gctaggtgca ctaggtactg 120
   gcattggcgg tatcacaacc tctactcagt tctactacaa actatctcaa gaactaaatg 180
   gggacatgga acgggtcgcc gactccctgg tcaccttgca agatcaactt aactccctag 240
   cagcagtagt ccttcaaaat cgaagagctt tagactcgct aaccgctgaa agagggggaa 300
   cctgtttatt tttaggggaa gaatgctgtt attatgttaa tcaatccgga atcgtcactg 360
   agaaagttaa agaaattcga gatcgaatac aacgtagagc agaagagctt cgaaacactg 420
   gaccctgggg cctcctcagc caatggatgc cctggattct ccccttctta ggacctctag 480
   cagctataat attgctactc ctctttggac cctgtatctt taacctcctt gttaactttg 540
   tctcttccag aatcgaagct gtaaaactac aaatggagcc caagatgcag tccaagacta 600
   agatctaccg cagacccctg gaccggcctg ctagcccacg atctgatgtt aatgacatca 660
   aaggcacccc tcctgaggaa atctcagctg cacaacctct actacgcccc aattcagcag 720
   gaagcagtta gagcggtcgt cggccaacct ccccaacagc acttaggttt tcctgttgag 780
   atgggggact gagagacagg actagctgga tttcctaggc tgactaagaa tccctaagcc 840
   tagctgggaa ggtgaccaca tccaccttta aacacggggc ttgcaactta gttcacacct 900
   gaccaatcag agagctcact aaaatgctaa ttaggcaaag acaggaggta aagaaatagc 960
   caatcatcta ttgcatgaga gcacagcagg agggacaatg atcgggatat aaacccaagt 1020
   cttcgagccg gcaacggcaa ccccctttgg gtcccctccc tttgtatggg agctctgttt 1080
   tcatgctatt tcactctatt aaatcttgca gctgcgaaaa aaaaaaaaaa aaaaaa 1136
<210> 26
   <211> 2782
   <212> ADN
   <213> Homo sapiens
<400> 26
   atgggagctg ttttcatgct atttcactct attaaatctt gcaactgcac tcttctggtc 60
   catgtttctt acggctcgag ctgagctttt gctcaccgtc caccactgct gtttgccacc 120
   accgcagacc tgccgctgac tcccatccct ctggatcctg cagggtgtcc gctgtgctcc 180
   tgatccagcg aagcgcccat tgccgctccc aattgggcta aaggcttgcc attgttcctg 240
   cacggctaag tgcctgggtt tgttctaatt gagctgaaca ctagtcactg ggttccatgg 300
   ttctcttctg tgacccacgg cttctaatag aactataaca cttaccacat ggcccaagat 360
   tccattcctt ggaatccgtg aggccaacga actccaggtc agagaatacg aagcttgcca 420
   ccatcttgga agcggcctgc taccatcttg gaagtggttc accaccatct tgggagctct 480
   gtgagcaagg accccccggt gacattttgg cgaccaccaa cggacatccc aagtgataca 540
   tcctgggaag gaccctaccc agtcatttta tctaccccaa ctgcggttaa agtggctgga 600
   gtggagtctt ggatacatca cacttgagtc aaatcctgga tactgccaaa ggaacctgaa 660
   aatccaggag acaacgctag ctattcctgt gaacctctag aggatttgcg cctgctcttc 720
   aaacaacaac caggaggaaa gtaactaaaa tcataaatcc ccatgggcct cccttatcat 780
   atttttctct gtagtgttct ttcaccctgt ttcactctca ctgcaccccc tccatgccgc 840
   tgtatgacca gtagctcccc tcacccagag tttctatgga gaatgcagcg tcccggaaat 900
   attgatgccc catcgtatag gagtctttct aagggaaccc ccaccttcac tgcccacacc 960
   catatgcccc gcaactgcta tcactctgcc actctttgca tgcatgcaaa tactcattat 1020
   tggacaggaa aaatgattaa tcctagttgt cctggaggac ttggagtcac tgtctgttgg 1080
   acttacttca cccaaactgg tatgtctgat gggggtggag ttcaagatca ggcaagagaa 1140
   aaacatgtaa aagaagtaat ctcccaactc accggggtac atggcacctc tagcccctac 1200
   aaaggactag atctctcaaa actacatgaa accctccgta cccatactcg cctggtaagc 1260
   ctatttaata ccaccctcac tgggctccat gaggtctcgg cccaaaaccc tactaactgt 1320
   tggatatgcc tccccctgaa cttcaggcca tatgtttcaa tccctgtacc tgaacaatgg 1380
   aacaacttca gcacagaaat aaacaccact tccgttttag taggacctct tgtttccaat 1440
   gtggaaataa cccatacctc aaacctcacc tgtgtaaaat ttagcaatac tacatacaca 1500
   accaactccc aatgcatcag gtgggtaact cctcccacac aaatagtctg cctaccctca 1560
   ggaatatttt ttgtctgtgg tacctcagcc tatcgttgtt tgaatggctc ttcagaatct 1620
   atgtgcttcc tctcattctt agtgccccct atgaccatct acactgaaca agatttatac 1680
   agttatgtca tatctaagcc ccgcaacaaa agagtaccca ttcttccttt tgttatagga 1740
   gcaggagtgc taggtgcact aggtactggc attggcggta tcacaacctc tactcagttc 1800
   tactacaaac tatctcaaga actaaatggg gacatggaac gggtcgccga ctccctggtc 1860
   accttgcaag atcaacttaa ctccctagca gcagtagtcc ttcgaaatcg aagagcttta 1920
   gacttgctaa ccgctgagag agggggaacc tgtttatttt taggggaaga atgctgttat 1980
   tatgttaatc aatccggaat cgtcactgag aaagttgaag aaattccaga tcgaatacaa 2040
   cgtatagcag aggagcttcg aaacactgga ccctggggcc tcctcagccg atggatgccc 2100
   tggattctcc ccttcttagg acctctagca gctataatat tgctactcct ctttggaccc 2160
   tgtatctttg acctccttgt taactttgtc tcttccagaa tcgaagctgt gaaactacaa 2220
   atggagccca agatgcagtc caagactaag atctaccgca gacccctgga ccggcctgct 2280
   agcccacgat ctgatgttaa tgacatcaaa ggcacccctc ctgaggaaat ctcagctgca 2340
   caacctctac tacgccccaa ttcagcagga agcagttaga gcggtggtcg gccaacctcc 2400
   ccaacagcac ttaggttttc ctgttgagat gggggactga gagacaggac tagctggatt 2460
   tcctaggctg actaagaatc cttaagccta ggtgggaagg tgaccacatc cacctttaaa 2520
   cacggggctt gcaacttagc tcacacctga ccaatcagag agctcactaa aatgctaatt 2580
   aggcaaagac aggaggtaaa gaaatagcca atcatttatt gcctgagagc acagcaggag 2640
   ggacaatgat cgggatataa acccaagttt tcgagccggc aacggcaacc ccctttgggt 2700
   cccctccctt tgtatgggag ctctgttttc atgctatttc actctattaa atcttgcaac 2760
   tgcaaaaaaa aaaaaaaaaa aa                                          2782
<210> 27
   <211> 666
   <212> ADN
   <213> Homo sapiens
<400> 27
   tgtccgctgt gctcctgatc cagcgaggcg cccattgccg ctcccaattg ggctaaaggc 60
   ttgccattgt tcctgcacgg ctaagtgcct gggtttgttc taattgagct gaacactant 120
   cactgggttc catggttctc ttctgtgacc cacggcttct aatataacta taacacttac 180
   cacatggccc aagattccat tccttggaat ccgtgaggcc aagaactcca ggtcagagaa 240
   tacgaggctt gccaccatct tggaagcggc ctgctaccat cttggaagtg gttcaccacc 300
   atcttgggag ctctgtgagc aaggaccccc cggtaacatt ttggcaacca cgaacggaca 360
   tccaaagtga atcgaagctg taaaactaca aatggagccc aagatgcagt ccaagactaa 420
   gatctaccgc agacccctgg accggcctgc tagcccacga tctgatgtta atgacatcaa 480
   aggcacccct cctgaggaaa tctcagctgc acaacctcta ctacgcccca attcagcagg 540
   aagcagttag agcggtcgtc ggccaacctc cccaacagca cttaggtttt cctgttgaga 600
   tgggggactg agagacagga ctagctggat ttcctaggct gactaagaat ccctaagcct 660
   agctgg                                                            666
<210> 28
   <211> 3372
   <212> ADN
   <213> Homo sapiens
<400> 28
   gacttcccaa ataccagagg aagcagagtg gtttacagtc ctggaccttc aggatgcctt 60
   cttctgcatc cctgtacatc ctgactctca attcttgttt gcctttgaag atacttcaaa 120
   cccagcatct caactcacct ggactatttt accccaaggg ttcagggata gtccccatct 180
   atttggccag gcattagccc aagacttgag ccaatcctca tacctggaca cttgtccttc 240
   ggtaggtgga tgatttactt ttggccgccc attcagaaac cttgtgccat caagccaccc 300
   aagcgctctt caatttcctc gctacctgtg gctacatggt ttccaaacca aaggctcaac 360
   tctgctcaca gcaggttact tagggctaaa attatccaaa ggcaccaggg ccctcagtga 420
   ggaacacatc cagcctatac tggcttatcc tcatcccaaa accctaaagc aactaagggg 480
   attccttggc gtaataggtt tctgccgaaa atggattccc aggtatggcg aaatagccag 540
   gtcattaaat acactaatta aggaaactca gaaagccaat acccatttag taagatggac 600
   aactgaagta gaagtggctt tccaggccct aacccaagcc ccagtgttaa gtttgccaac 660
   agggcaagac ttttgttcat atgtcacaga aaaaacagga atagctctag gagtccttac 720
   acagatccga gggatgagct tgcaacctgt ggcacacctg actaaggaaa ttgatgtagt 780
   ggcaaagggt tgacctcatt gtttacgggt agtggtggca gtagcagtct tagtatctga 840
   agcagttaaa ataatacagg gaagagatct tactgtgtgg acatctcatg atgtgaatgg 900
   catactcact gctaaaggag acttgtggct gtcagacaac tgtttactta aatgtcaggc 960
   tctattactt gaagggccag tgctgcgact gtgcacttgt gcaactctta acccagccac 1020
   atttcttcca gacaatgaag aaaagataaa acataactgt caacaagtaa tttctcaaac 1080
   ctatgccact cgaggggacc ttttagaggt tcctttgact gatcccgacc tcaacttgta 1140
   tactgatgga agttcctttg tagaaaaagg acttcgaaaa gtggggtatg cagtggtcag 1200
   tgataatgga atacttgaaa gtaatcccct cactccagga actagtgctc agctagcaga 1260
   actaatagcc ctcacttggg cactagaatt aggagaagaa aaaagggcaa atataataca 1320
   gactctaaat atgcttacct agtcctccat gcccatgcag caatatggaa agaaagggaa 1380
   ttcctaactt ctgagagaac acctatcaaa catcaggaag ccattaggaa attattattg 1440
   gctgtacaga aacctagaga ggtggcagtc ttacactgcc ggggtcatca caaaggaaag 1500
   gaaagggaaa tacaagagaa ctgccaagca tatattgaag ccaaaagagc tgcaaggcag 1560
   gaccctccat tagaaatgct tattaaactt cccttagtat agggtaatcc cttccgggaa 1620
   accaagcccc agtactcagc aggagaaaca gaatggggaa cctcacgagg cagttttctc 1680
   ccctcgggac ggttagccac tgaagaaggg aaaatacttt tgcctgcaac tatccaatgg 1740
   aaattactta aaacccttca tcaaaccttt cacttaggca tcgatagcac ccatcagatg 1800
   gccaaatcat tatttactgg accaggcctt ttcaaaacta tcaagcagat agtcagggcc 1860
   tgtgaagtgt gccagagaaa taatcccctg ccttatcgcc aagctccttc aggagaacaa 1920
   agaacaggcc attaccctgg agaagactgg caactgattt tacccacaag cccaaacctc 1980
   agggatttca gtatctacta gtctgggtag atactttcac gggttgggca gaggccttcc 2040
   cctgtaggac agaaaaggcc caagaggtaa taaaggcact agttcatgaa ataattccca 2100
   gattcggact tccccgaggc ttacagagtg acaatagccc tgctttccag gccacagtaa 2160
   cccagggagt atcccaggcg ttaggtatac gatatcactt acactgcgcc tgaaggccac 2220
   agtcctcagg gaaggtcgag aaaatgaatg aaacactcaa aggacatcta aaaaagcaaa 2280
   cccaggaaac ccacctcaca tggcctgttc tgttgcctat agccttaaaa agaatctgca 2340
   actttcccca aaaagcagga cttagcccat acgaaatgct gtatggaagg cccttcataa 2400
   ccaatgacct tgtgcttgac ccaagacagc caacttagtt gcagacatca cctccttagc 2460
   caaatatcaa caagttctta aaacattaca aggaacctat ccctgagaag aggaaaagaa 2520
   tattccaccc aagtgacatg gtattagtca agtcccttcc ctctaattcc ccatccctag 2580
   atacatcctg ggaaggaccc tacccagtca ttttatctac cccaactgcg gttaaagtgg 2640
   ctggagtgga gtcttggata catcacactt gagtcaaatc ctggatactg ccaaaggaac 2700
   ctgaaaatcc aggagacaac gctagctatt cctgtgaacc tctagaggat ttgcgcctgc 2760
   tcttcaaaca acaaccagga ggaaaaatcg aagctgtaaa actacaaatg gagcccaaga 2820
   tgcagtccaa gactaagatc taccgcagac ccctggaccg gcctgttagc ccacgatctg 2880
   atgttaatga catcaaaggc acccctcctg aggaaatctc agctgcacaa cctctactac 2940
   gccccaattc agcaggaagc agttagagcg gtcgtcggcc aacctcccca acagcactta 3000
   ggttttcctg ttgagatggg ggactgagag acaggactag ctggatttcc taggctgatt 3060
   aagaatccct aagcctagct gggaaggtga ccacatccac ctttaaacac ggggcttgca 3120
   acttagctca cacctgacca atcagagagc tcactaaaat gctaattagg caaagacagg 3180
   aggtaaagaa atagccaatc atttattgcc tgagagcaca gcaggaggga caatgatcgg 3240
   gatataaacc caagttttcg agccggcaac ggcaaccccc tttgggtccc ctccctttgt 3300
   atgggagctc tgttttcatg ctatttcact ctattaaatc ttgcaactgc aaaaaaaaaa 3360
   aaaaaaaaaa aa                                                     3372
<210> 29
   <211> 2372
   <212> ADN
   <213> Homo sapiens
<400> 29
   actgcactct tctggtccat gtttcttacg gctcgagctg agcttttgct caccgtccac 60
   cactgctgtt tgccaccacc gcagacctgc cgctgactcc catccctctg gatcctgcag 120
   ggtgtccgct gtgctcctga tccagcgagg cgcccattgc cgctcccaat tgggctaaag 180
   gcttgccatt gttcctgcac ggctaagtgc ctgggtttgt tctaattgag ctgaacacta 240
   atcactgggt tccatggttc tcttctgtga cccacggctt ctaatagaac tataacactt 300
   accacatggc ccaagattcc attccttgga atccgtgagg ccaagaactc caggtcagag 360
   aatacgaggc ttgccaccat cttggaagcg gcctgctacc gtcttggaag tggttcacca 420
   ccatcttggg agctctgtga gcaaggaccc cccggtaaca ttttggcaac caacgacgga 480
   catccaaagt gatgggaaac gttccccgca agacaaaaac gcccctaaga cgtattctgg 540
   agaattggga ccaatttgac cctcagacac taagaaagaa acgacttata ttcttctgca 600
   gtgccgcctg gcactcctga gggaagtata aattataaca ccatcttaca gctagacctc 660
   ttttgtagaa aaggcaaatg gagtgaagtg ccataagtac aaactttctt ttcattaaga 720
   gacaactcac aattatgtaa aaagtgtgat ttatgcccta caggaagcct tcagagtcta 780
   cctccctatc ccagcatccc cgactccttc cccaactaat aaggaccccc cttcaaccca 840
   aatggtccaa aaggagatag acaaaagggt aaacagtgaa ccaaagagtg ccaatattcc 900
   ccaattatga cccctccaag cagtgggagg aagagaattc ggcccagcca gagtgcatgt 960
   gcctttttct ctcccagact taaagcaaat aaaaacagac ttaggtaaat tctcagataa 1020
   ccctgatggc tatattgatg ttttacaagg gttaggacaa ttctttgatc tgacatggag 1080
   agatataatg tcactgctaa atcagacact aaccccaaat gagagaagtg ccaccataac 1140
   tgcagcctga gggtttggcg tctctggtat ctcagtcagg tcaatggata nggatgacaa 1200
   cagaaggaaa ganaatgatt ccccacaggc cagcaggcag ttcccagtct agaccctcat 1260
   tgggacacag aatcagaaca tggagattgg tgctgcagac atttgctaac ttgtgtgcta 1320
   gaaggactaa ggaaaactag gaagaagtct atgaattact caatgatgtc caccataaca 1380
   cagggaaggg aagaaaatcc tactgccttt ctggagagac taagggaggc attgaggaag 1440
   cgtgcctctc tgtcacctga ctcttctgaa ggccaactaa tcttaaagcg taagtttatc 1500
   actcagtcag ctgcagacat tagaaaaaac ttcaaaagtc tgccgtaggc ccggagcaaa 1560
   acttagaaac cctattgaac ttggcaacct cggtttttta taatagagat caggaggagc 1620
   aggcggaaca ggacaaacgg gattaaaaaa aaggccaccg ctttagtcat gaccctcagg 1680
   caagtggact ttggaggctc tggaaaaggg aaaagctggg caaattgaat gcctaatagg 1740
   gcttgcttcc agtgcggtct acaaggacac tttaaaaaag attgtccaag tagaagtaag 1800
   ccgccccttc gtccatgccc cttatttcaa gggaatcact ggaaggccca ctgccccagg 1860
   ggacaaaggt cttttgagtc agaagccact aaccagatga tccagcagca ggactgaggg 1920
   tgcctggggc aagcgccatc ccatgccatc accctcacag agccctgggt atgcttgacc 1980
   attgagggcc aggaaggttg tctcctggac actggtgcgg tcttcttagt cttactcttc 2040
   tgtcccggac aactgtcctc cagatctgtc actattctga gggggtccnt aagacgggca 2100
   gtcactagat actttttccc agccactaag ttatgaactg gggagcttta ttcttttcac 2160
   atgcttttct aattatgctt gaaagcccca ctaccttgtt agggagagac attctagcaa 2220
   aagcaggggc cattatacac ctgaacatag gagaaggaac acccgtttgt tgtncccctg 2280
   cttgaggaag gaattaatcc tgaagtctgg gcaacagaag gacaatatgg acgagccaaa 2340
   gaatgcccgt cctgttcaag ttaaactaaa gg                               2372
<210> 30
   <211> 7582
   <212> ADN
   <213> Homo sapiens
<400> 30
   caacaatcgg gatataaacc caggcattcg agctggcaac agcagccccc ctttgggtcc 60
   cttccctttg tatgggagct gttttcatgc tatttcactc tattaaatct tgcaactgca 120
   ctcttctggt ccatgtttct tacggctcga gctgagcttt tgctcaccgt ccaccactgc 180
   tgtttgccac caccgcanac ctgccgctga ctcccatccc tctggatcct gcagggtgtc 240
   cgctgtgctc ctgatccagc gargcgccca ttgccgctcc caattgggct aaaggcttgc 300
   cattgtncct gcacggctaa gtgcctgggt ttgttctaat tgagctgaac actantcact 360
   gggttccatg gttctcttct gtgacccacg gcttctaata kaactataac acttaccaca 420
   tggcccaaga ttccattcct tggaatccgt gaggscaacg aactccaggt cagagaatac 480
   gargcttgcc accatcttgg aagcggcctg ctaccrtctt ggaagtggtt caccaccatc 540
   ttgggagctc tgtgagcaag gaccccccgg tracattttg gcraccamsr acggacatcc 600
   maagtgatgg gaaacgttcc ccgcaagaca aaaacgcccc taagacgtat tctggaraat 660
   tgggamcaat ttgaccctca gacactaaga aagaaacgac ttatattctt ctgcagtgcc 720
   gcctggcact cctgagggaa gtataaatta taacaccatc ttacagctag acytcttttg 780
   tagaaaaggc aaatggagtg aagtgccata agtacaaact ttcttttcat taagagacaa 840
   ctcacaatta tgtaaaaagt gtgatttatg ccctacagga agccttcaga gtctacctcc 900
   ctatcccagc atccccgact ccttccccam ytaataagga ccccccttca acccaaatgg 960
   tccaaaagga gatagacaaa agggtaaaca gtgaaccaaa gagtgccaat attccccaat 1020
   tatgacccct cccaagcagt gggaggaaga gaattcggcc cagccagagt gcatgtgcyt 1080
   tttyytctcc cagacttaaa gcaaataaaa acagacttag gtaaattctc agataaycct 1140
   gatggctata ttgrtgtttt acaagggtta ggacaattct ttgatctgac atggagagat 1200
   atatatgtca ctgctaaatc agacactaac cccaaatgag agaagtgcca ccataactgc 1260
   agcctgagrg tttggcgatc tctggtatct cagtcaggtc aatggatang gatgacaaca 1320
   gaaggaaaga naatgattcc ccacaggcca gcargcagtt cccagtctas accctcattg 1380
   gggacacaga aatcagtaac atgggagatt ggtgctgcag acatttgcta acttgtgtgc 1440
   tasaaggact aaggaaaact asgaagaaar tctaygaatt actcaatgat gtccaccata 1500
   acacagggga agggaagaaa atcctactgc ctttctggag agactaaggg aggcattgag 1560
   gaagcgtgcc tctctgtcac ctgactcttc tgaaggccaa ctaatcttaa agcgtaagtt 1620
   tatcactcag tcagctgcag acattagaaa aaacttcaaa agtctgccgt aggcccggag 1680
   caaaacttag aaaccctatt gaacttggca acytcggttt tttataatag agatcaggag 1740
   gagcaggcgg aacaggacaa acgggattaa aaaaaaggcc accgctttag tcatgaccct 1800
   caggcaagtg gactttggag gctctggaaa agggaaaagc tgggcaaatt gaatgcctaa 1860
   tagggcttgc ttccagtgcg gtctacaagg acactttaaa aaagattgtc caagtagaag 1920
   taagccgccc cttcgtccat gccccttatt tcaagggaat cactggaagg cccactgccc 1980
   caggggacaa aggtcttttg agtcagaagc cactaaccag atgatccagc agcaggactg 2040
   agggtgcctg gggcaagcgc catcccatgc catcaccctc acagagccct gggtatgctt 2100
   gaccattgag ggccaggaag gttgtctcct ggacactggt gcggtcttct tagtcttact 2160
   cttctgtccc ggacaactgt cctccagatc tgtcactatt ctgagggggt ccntaagacg 2220
   ggcagtcact agatacttty tcccagccac taagttatga actggggagc tttattcttt 2280
   tcacatgctt ttctaattat gcttgaaagc cccactacct tgttagggag agacattcta 2340
   gcaaaagcag gggccattat acacctgaac ataggagaag gaacacccgt ttgttgtncc 2400
   cctgcttgag gaaggaatta atcctgaagt ctgggcaaca gaaggacaat atggacgagc 2460
   caaagaatgc ccgtcctgtt caagttaaac taaaggattc cacttccttt ccctaccaaa 2520
   ggcagtaccc cctcagaccc aaggcccaac aaggattcca aaagattgtt aaggacttaa 2580
   aagcccaagg cttagtaaaa ccatgcataa ctccctgcag taattccgta gtggattgag 2640
   gaggcacaga aacccagtgg acagtggagg gttagtgcaa gatctcagga ttatcaatgg 2700
   aggccgttgt ccttttatac ccagctgtac ctagccctta tactgtgmyt tcccaaatac 2760
   cagaggaagc agagtggttt acastcctgg accttmagga tgccttcttc tgcatccctg 2820
   tacatcctga ctctcaattc ttgtttgcct ttgaagatac ttcaaaccca rcatctcaac 2880
   tcacctggac trttttaccc caagggttca gggatagycc ccatctattt ggccaggcat 2940
   tagcccaaga cttgagycar tymtcatacc tggacactct tgtccttcrg takgtggatg 3000
   atttactttt rgcygccyrt tcagaaacct tgtgccatca agccacccaa gcrctcttma 3060
   atttcctcgc yacctgtggc tacawggttt ccaaacsara rgctcarctc tgctcacagc 3120
   aggttaaata cttaggrcta arattatcca aaggcaccar ggccctcagt gaggaayrya 3180
   tccagcctat actggcttat cctcatcyca aaaccctaaa gcaactaagr grrttccttg 3240
   gcrtaayagg yttctgccga awatggattc cccaggtwtg gcraaatagc caggycatta 3300
   watacastaa ttaaggaaac tcagaaagcc aatacccatt tartaagatg gayamctgaa 3360
   gymraagtgg ctttccaggc ccctaaagaa ggccttaaac ccaagyccca gtgttaagyt 3420
   tgccaacrgg gcaagacttt tsttyatayr tcacagaaaa aaacagraay agctctrgga 3480
   gtccttacac agrtccragg gaygagcttg caaccyrtgg cryacctgas taaggaaayt 3540
   gatgtagtgg caaagggttg rcytcattgt ttaygggtag tggtggcagt agcagtykta 3600
   gtatctgaag cagttaaaat aatacagggr agagatctta ctgtgtggac atctcatgak 3660
   gtgaayrgca tactcactgc taaaggagac ttgtggctgt cagacaacyg tttacttaaa 3720
   trtcaggctc tattacttga arggccagtg ctgcractgt gcacttgtgc aactcttaac 3780
   ccagycncat ttcttccaga caatgaagaa aagataraay ataactgtca acaartaatt 3840
   tctcaaacct atgccactcg aggggacctt ytagargttc cyttgactga tccygacctt 3900
   caacttgtat actgatggaa gttcctttgt agaaaaagga cttcgaaaag yggggtatgc 3960
   agtggtcagt gataatggaa tayttgaaag taatcccctc actccaggaa ctagtgctya 4020
   gctrgcagaa ctaatagccy tcaytkgggc actagaatta ggagaagraa aaagggyaaa 4080
   tatatataca gactctrart atgctyacct agtcntccat gcccatgmrg caatatgsar 4140
   agaaagggaa ttcctaactt cygagrgaac acctatcama catcaggaag ccattaggar 4200
   attattaytg gcwgtacaga aacctaraga ggtggmagtc ttacactgcy ggggtcatca 4260
   naaaggaaag raaagggaaa tasaagrgaa ytgccaagca katattgaag cmaaaagagc 4320
   tgcaaggcag gaccctccat tagaaatgct tattaaactt cccttagtat agggtaatcc 4380
   cttccgggaa accaagcccc agtactcagc aggagaaaca gaatggggaa cctcacgagg 4440
   cagttttctc ccctcgggac ggttagccac tgaagaaggg aaaatacttt tgcctgcaac 4500
   tatccaatgg aaattactta aaacccttca tcaaaccttt cacttaggca tcgatagcac 4560
   ccatcaratg gccaaatcat tatttactgg accaggcctt ttcaaaacta tcaagcarat 4620
   aktcagggcc tgtgaaktgt gccararaaa taatcccctg cctyatcgcc aagctccttc 4680
   aggaraacaa araacaggcc attaccctgr araaractgg caactgattt tacccacaag 4740
   cccaaacctc agggatttca gtatctacta gtctgggtar atactttcac gggttgggca 4800
   raggccttcc cctgtaggac agaaaaggcc caagaggtaa taaaggcact agttcatgaa 4860
   ataattccca gattcggact tccccgaggc ttacagagtg acaatagccc tgctttccag 4920
   gccacagtaa cccagggagt atcccaggcg ttaggtatac gatatcactt acactgcgcc 4980
   tgaaggccac agtcctcagg gaaggtcgag aaaatgaatg aaayactcaa aggacatcta 5040
   aaaaagcaaa cccaggaaac ccacctcaca tggcctgytc tgttgcctat agccttaaaa 5100
   agaatctgca actttcccca aaaagcagga cttagcccat acgaaatgct gtatggaagg 5160
   cccttcataa ccaatgacct tgtgcttgac ccaagacagc caacttagtt gcagacatca 5220
   cctccttagc caaatatcaa caagttctta aaacattaca aggaacctat ccctgagaag 5280
   agggaaaaga actattccac ccwwgtgaca tggtattagt caagtccctt cyctctaatt 5340
   ccccatccct agatacatcc tgggaaggac cctacccagt cattttatyt accccaactg 5400
   cggttaaagt ggctggagtg gagtcttgga tacatcacac ttgagtcaaa tcctggatac 5460
   tgccaaagga acctgaaaat ccaggagaca acgctagcta ttcctgtgaa cctctagagg 5520
   atttgcgcct gctcttcaaa caacaaccag gaggaaagta actaaaatca taaatccccc 5580
   atggscctcc cttatcatat ttttctctkt astgttsttt yaccctsttt cactctcact 5640
   gcaccccctc catgccgctg tatgaccagt agctccccty accmagagtt tctatggaga 5700
   atgcagcgtc ccggaaatat tgatgcccca tcgtatagga gtctttstaa gggaaccccc 5760
   accttcactg cccacaccca tatgccccgc aactgctatc actctgccac tctttgcatg 5820
   catgcaaata ctcattattg gacaggaaaa atgattaatc ctagttgtcc tggaggactt 5880
   ggagtcactg tctgttggac ttacttcacc caaactggta tgtctgatgg gggtggagtt 5940
   caagatcagg caagagaaaa acatgtaaaa gaagtaatct cccaactcac csgggtacat 6000
   ggcacctcta gcccctacaa aggactagat ctctcaaaac tacatgaaac cctccgtacc 6060
   catactcgcc tggtaagcct atttaatacc accctcactg ggctccatga ggtctcggcc 6120
   caaaacccta ctaactgttg gatatgcctc cccctgaact tcargccata tgtttcaatc 6180
   cctgtacctg aacaatggaa caacttcagc acagaaataa acaccacttc cgttttagta 6240
   ggacctcttg tttccaatst ggaaataacc catacctcaa acctcacctg tgtaaaattt 6300
   agcaatacta catacacaac caactcccaa tgcatcaggt gggtaactcc tcccacacaa 6360
   atagtctgcc taccctcagg aatatttttt gtctgtggta cctcagccta tcgttgtttg 6420
   aatggctctt cagaatctat gtgcttcctc tcattcttag tgcccccyat grccatctac 6480
   actgaacaag atttatacag ttatgtcata tctaagcccc gcaacaaaag agtacccatt 6540
   cttccttttg ttataggagc aggagtgcta ggtgcactag gtactggcat tggcggtatc 6600
   acaacctcta ctcagttcta ctacaaacta tctcaagaac taaatgggga catggaacgg 6660
   gtcgccgact ccctggtcac cttgcaagat caacttaact ccctagcagc agtagtcctt 6720
   craaatcgaa gagctttaga ctygctaacc gctgaragag ggggaacctg tttattttta 6780
   ggggaagaat gctgttatta tgttaatcaa tccggaatcg tcactgagaa agttraagaa 6840
   attcsagatc gaatacaacg takagcagar gagcttcgaa acactggacc ctggggcctc 6900
   ctcagccrat ggatgccctg gattctcccc ttcttaggac ctctagcagc tataatattg 6960
   ctactcctct ttggaccctg tatctttrac ctccttgtta actttgtctc ttccagaatc 7020
   gaagctgtra aactacaaat ggagcccaag atgcagtcca agactaagat ctaccgcaga 7080
   cccctggacc ggcctgytag cccacgatct gatgttaatg acatcaaagg cacccctcct 7140
   gaggaaatct cagctgcaca acctctacta cgccccaatt cagcaggaag cagttagagc 7200
   ggtsgtcggc caacctcccc aacagcactt aggttttcct gttgagatgg gggactgaga 7260
   gacaggacta gctggatttc ctaggctgay taagaatccy taagcctags tgggaaggtg 7320
   accacatcca cctttaaaca cggggcttgc aacttagytc acacctgacc aatcagagag 7380
   ctcactaaaa tgctaattag gcaaagacag gaggtaaaga aatagccaat catytattgc 7440
   mtgagagcac agcaggaggg acaatgatcg ggatataaac ccaagtyttc gagccggcaa 7500
   cggcaacccc ctttgggtcc cctccctttg tatgggagct ctgttttcat gctatttcac 7560
   tctattaaat cttgcarctg cr 7582
<210> 31
   <211> 363
   <212> PRT
   <213> Homo sapiens
<400> 31
   Met Gly Asn Ile Pro Pro Lys Ala Lys Thr Pro Leu Arg Cys Ile Leu
   1               5                   10                  15
   Glu Asn Trp Asp Gln Cys Asp Thr Gln Thr Leu Arg Lys Lys Arg Phe
               20                  25                  30
   Ile Phe Phe Cys Ser Thr Ala Trp Pro Gln Tyr Pro Leu Gln Gly Arg
           35                  40                  45
   Glu Thr Trp Leu Pro Glu Gly Ser Ile Asn Tyr Asn Ile Ile Leu Gln
       50                  55                  60
   Leu Asp Leu Phe Cys Arg Lys Glu Gly Lys Trp Ser Glu Val Pro Tyr
   65                  70                  75                  80
   Val Gln Thr Phe Phe Ser Leu Arg Asp Asn Ser Gln Leu Cys Lys Lys
                   85                  90                  95
   Cys Gly Leu Cys Pro Thr Gly Ser Pro Gln Ser Pro Pro Pro Tyr Pro
               100                 105                 110
   Ser Val Pro Pro Pro Thr Pro Ser Ser Thr Asn Lys Asp Pro Pro Leu
           115                 120                 125
   Thr Gln Thr Val Gln Lys Glu Ile Asp Lys Gly Val Asn Asn Glu Pro
       130                 135                 140
   Lys Ser Ala Asn Ile Pro Arg Leu Cys Pro Leu Gln Ala Val Arg Gly
   145                 150                 155                 160
   Gly Glu Phe Gly Pro Ala Arg Val Pro Val Pro Phe Ser Leu Ser Asp
                   165                 170                 175
   Leu Lys Gln Ile Lys Ile Asp Leu Gly Lys Phe Ser Asp Asn Pro Asp
               180                 185                 190
   Gly Tyr Ile Asp Val Leu Gln Gly Leu Gly Gln Ser Phe Asp Leu Thr
           195                 200                 205
   Trp Arg Asp Ile Met Leu Leu Leu Asn Gln Thr Leu Thr Pro Asn Glu
       210                 215                 220
   Arg Ser Ala Ala Val Thr Ala Ala Arg Glu Phe Gly Asp Leu Trp Tyr
   225                 230                 235                 240
   Leu Ser Gln Val Asn Asn Arg Met Thr Thr Glu Glu Arg Thr Thr Pro
                   245                 250                 255
   Thr Gly Gln Gln Ala Val Pro Ser Val Asp Pro His Trp Asp Thr Glu
                       260                 265                 270
   Ser Glu His Gly Asp Trp Cys His Lys His Leu Leu Thr Cys Val Leu
           275                 280                 285
   Glu Gly Leu Arg Lys Thr Arg Lys Lys Pro Met Asn Tyr Ser Met Met
       290                 295                 300
   Ser Thr Ile Thr Gln Gly Lys Glu Glu Asn Pro Thr Ala Phe Leu Asp
   305                 310                 315                 320
   Arg Leu Arg Glu Ala Leu Arg Lys His Thr Ser Leu Ser Pro Asp Ser
                   325                 330                 335
   Ile Glu Gly Gln Leu Ile Leu Lys Asp Lys Phe Ile Thr Gln Ser Ala
               340                 345                 350
   Ala Asp Ile Arg Lys Asn Phe Lys Ser Leu Pro
           355                 360